# EUROPEAN PATENT SPECIFICATION

(11) **EP 2 854 822 B1**
(45) Date of publication and mention of the grant of the patent: **22.07.2020**
(21) Application number: 13798089.2
(22) Date of filing: 30.05.2013
(51) Int. Cl.: A61K 31/715, A61P 31/04, A61P 31/10, G01N 33/53, G01N 33/569

(54) **POLYSACCHARIDE COMPOSITIONS AND METHODS OF USE**
POLYSACCHARIDZUSAMMENSETZUNGEN UND VERFAHREN ZUR VERWENDUNG
COMPOSITIONS DE POLYSACCHARIDE ET PROCÉDÉS D'UTILISATION

(30) Priority: 30.05.2012 US 201261653389 P; 26.05.2013 US 201361827661 P
(43) Date of publication of application: 08.04.2015
(73) Proprietor: The Brigham and Women's Hospital, Inc., Boston, MA 02115 (US)
(72) Inventor: PIER, Gerald, B., Brookline, MA 02446 (US); CYWES-BENTLEY, Colette, Brookline, MA 02445 (US); SKURNIK, David, 75015 Paris (FR)
(74) Representative: Graham Watt & Co LLP
(86) International application number: PCT/US2013/043283
(87) International publication number: WO 2013/181348

(56) References cited:
- WO-A2-2004/043405
- WO-A2-2004/043405
- WO-A2-2005/103084
- MAIRA-LITRAN T ET AL: "COMPARATIVE OPSONIC AND PROTECTIVE ACTIVITIES OF STAPHYLOCOCCUS AUREUS CONJUGATE VACCINES CONTAINING NATIVE OR DEACETYLATED STAPHYLOCOCCAL POLY-N-ACETYL-BETA-(1-6)-GLUCOSAMINE", INFECTION AND IMMUNITY, AMERICAN SOCIETY FOR MICROBIOLOGY, US, vol. 73, no. 10, 1 October 2005 (2005-10-01), pages 6752-6762, XP009058830, ISSN: 0019-9567, DOI: 10.1128/IAI.73.10.6752-6762.2005
- M. L. GENING ET AL: "Synthetic -(1->6)-Linked N-Acetylated and Nonacetylated Oligoglucosamines Used To Produce Conjugate Vaccines for Bacterial Pathogens", INFECTION AND IMMUNITY, vol. 78, no. 2, 1 February 2010 (2010-02-01), pages 764-772, XP055006302, ISSN: 0019-9567, DOI: 10.1128/IAI.01093-09
- N. CERCA ET AL: "Protection against Escherichia coli infection by antibody to the Staphylococcus aureus poly-N-acetylglucosamine surface polysaccharide", PROCEEDINGS OF THE NATIONAL ACADEMY OF SCIENCES, vol. 104, no. 18, 1 May 2007 (2007-05-01), pages 7528-7533, XP055098339, ISSN: 0027-8424, DOI: 10.1073/pnas.0700630104
- DAVID SKURNIK ET AL: "Targeting Pan-Resistant Bacteria With Antibodies to a Broadly Conserved Surface Polysaccharide Expressed During Infection", JOURNAL OF INFECTIOUS DISEASES. JID, UNIVERSITY OF CHICAGO PRESS, CHICAGO, IL , vol. 205, no. 11 23 March 2012 (2012-03-23), pages 1709-1718, XP002711313, ISSN: 0022-1899, DOI: 10.1093/INFDIS/JIS254 Retrieved from the Internet: URL:http://jid.oxfordjournals.org/content/ 205/11/1709 [retrieved on 2012-03-23]
- C. CYWES-BENTLEY ET AL: "Antibody to a conserved antigenic target is protective against diverse prokaryotic and eukaryotic pathogens", PROCEEDINGS OF THE NATIONAL ACADEMY OF SCIENCES, vol. 110, no. 24, 11 June 2013 (2013-06-11), pages E2209-E2218, XP055222814, US ISSN: 0027-8424, DOI: 10.1073/pnas.1303573110
- GENING, MARINA L. ET AL.: 'Synthetic beta-(I-j6)-Linked N-Acetylated and Nonacetylated Oligoglucosamines Used To Produce Conjugate Vaccines for Bacterial Pathogens' INFECTION AND IMMUNITY. vol. 78, no. 2, February 2010, pages 764 - 772, XP055006302
- Alexander G. Bobrov ET AL: "Insights into Yersinia pestis biofilm development: topology and co-interaction of Hms inner membrane proteins involved in exopolysaccharide production", Environmental Microbiology, vol. 10, no. 6, 14 June 2008 (2008-06-14), pages 1419-1432, XP055345705, GB ISSN: 1462-2912, DOI: 10.1111/j.1462-2920.2007.01554.x
- G. Parise ET AL: "Role of a Putative Polysaccharide Locus in Bordetella Biofilm Development", Journal of Bacteriology, vol. 189, no. 3, 1 February 2007 (2007-02-01), pages 750-760, XP055345700, US ISSN: 0021-9193, DOI: 10.1128/JB.00953-06
- Matt S. Conover ET AL: "The Bps polysaccharide of Bordetella pertussis promotes colonization and biofilm formation in the nose by functioning as an adhesin", MOLECULAR MICROBIOLOGY., vol. 77, no. 6, 1 September 2010 (2010-09-01), pages 1439-1455, XP055346089, GB ISSN: 0950-382X, DOI: 10.1111/j.1365-2958.2010.07297.x
- J. B. Kaplan ET AL: "Genes Involved in the Synthesis and Degradation of Matrix Polysaccharide in Actinobacillus actinomycetemcomitans and Actinobacillus pleuropneumoniae Biofilms", Journal of Bacteriology, vol. 186, no. 24, 15 December 2004 (2004-12-15), pages 8213-8220, XP055568931, US ISSN: 0021-9193, DOI: 10.1128/JB.186.24.8213-8220.2004
- X. Wang ET AL: "The pgaABCD Locus of Escherichia coli Promotes the Synthesis of a Polysaccharide Adhesin Required for Biofilm Formation", Journal of Bacteriology, vol. 186, no. 9, 1 May 2004 (2004-05-01), pages 2724-2734, XP055568590, US ISSN: 0021-9193, DOI: 10.1128/JB.186.9.2724-2734.2004
- DATABASE MEDLINE [Online] US NATIONAL LIBRARY OF MEDICINE (NLM), BETHESDA, MD, US; 2008, HINNEBUSCH B J ET AL: "Yersinia pestis biofilm in the flea vector and its role in the transmission of plague.", Database accession no. NLM18453279 & CURRENT TOPICS IN MICROBIOLOGY AND IMMUNOLOGY 2008, vol. 322, 2008, pages 229-248, ISSN: 0070-217X

## Description

### FIELD OF INVENTION

The present invention relates to the use of certain polysaccharide antigen and antibody compositions in the detection, prevention and/or treatment of infections by particular pathogens.

### BACKGROUND OF INVENTION

Developing immunotherapies for various infections, whether bacterial, viral, fungal or parasitic in nature, is a high priority. Many current immunotherapies target particular microbial species via species-specific antigens. Less common are immunotherapies that target and are effective against a broad range of microbes.

*Staphylococcus aureus* and *S. epidermidis* express a poly-N-acetyl glucosamine (PNAG) polysaccharide antigen at their surface. PNAG is synthesized *in vivo* by the gene products of the *ica* gene locus in these bacteria. Similarly, *Escherichia coli* and other gram-negative bacteria contain a homologous genetic locus termed the *pga* locus which also encodes synthesis of the proteins that may be used to synthesize PNAG. Thus, bacteria with an intact *ica* or *pga* locus can produce PNAG. It was previously found that deacetylated forms of this antigen was particularly effective at stimulating antigen-specific immune responses characterized in part by the induction of opsonic antibodies.

### SUMMARY OF INVENTION

The invention relates to pharmaceutical compositions for use according to the claims. The invention is based, in part, on the unexpected and surprising finding that the polysaccharide poly N-acetyl glucosamine (PNAG) is expressed by a number and a variety of pathogens that were previously not known or suggested to express this polysaccharide. The invention therefore provides compositions comprising this polysaccharide or antibodies specific for this polysaccharide for use in preventing and/or treating infections by these particular pathogens, and optionally treating and/or preventing a disease or disorder that may result from such infection.

Surprisingly, the pathogens found to express PNAG, in accordance with the invention, range over a number of classes and types. These classes and specific pathogens are as follows: (a) gram-positive cocci: vaccine and non-vaccine strains of *Streptococcus pneumoniae*, Group A *Streptococcus* such as *Streptococcus pyogenes*, Group B *Streptococcus* such as *Streptococcus agalactiae*, and Group C *Streptococcus* such as *Streptococcus dysagalactiae*, and *Enterococcus faecalis*; (b) gram-positive rods: *Listeria monocytogenes*, *Clostridium difficile*, *Bacillus subtilis*, *Mycobacterium tuberculosis*, and *M. smegmatis*; (c) gram-negative cocci and coccobacilli: *Neisseria meningitides*, *N. gonorrhoeae*, Non-typable *Hemophilus influenzae*, *Hemophilus ducreyi*, *Helicobacter pylori*, and *Campylobacter jejuni*; (d) gram-negative rods: *Bacteroides fragilis, B. thetaiotamicron, B vulgatis, Citrobacter rodentium*, *Vibrio cholerae*, *Salmonella enterica* serovar typhi and *Salmonella enterica* serovar typhimurium; (e) fungi: *Candida albicans* (yeast), *Candida albicans* (hyphae), *Aspergillus flavus*, *Fusarium spp* such as *Fusarium solani*, and *Cryptococcus neoformans*; and (f) parasites: *Plasmodium bergei* and *P. falciparum* (including sporozoites); and *Trichomonas vaginalis* (*T. vaginalis*). PNAG expression by these pathogens is particularly surprising since none of them have an identifiable genetic locus related to the *ica* locus of *Staphylococci* or a *pga* locus of *E. coli*, which encode proteins involved in PNAG and related polysaccharide synthesis in certain bacteria. These pathogens are therefore referred to herein as non-*ica*/*pga* pathogens to indicate that they do not have identifiable *ica* or *pga* loci.

Methods are described for detecting any of the foregoing pathogens using for example antibodies specific for PNAG.

Thus, in one aspect, a method is described comprising administering to a subject having or at risk of developing an infection by a non-*ica*/*pga* but PNAG-positive pathogen an effective amount for inducing an immune response against the pathogen of an isolated polysaccharide having the formula 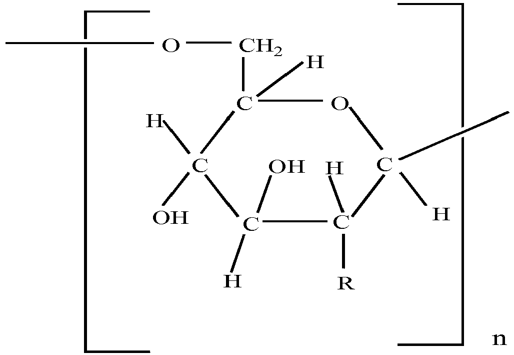 wherein n is at least 5, R is selected from the group consisting of -NH-CO-CH₃ and -NH₂, provided that less than 50% of the R groups are -NH-CO-CH₃.

There is also described a method comprising administering to a subject having or at risk of developing an infection by a non-*ica*/*pga* PNAG-positive pathogen an effective amount for inducing an immune response against the pathogen of an isolated polysaccharide conjugated to a carrier, wherein the polysaccharide has the formula 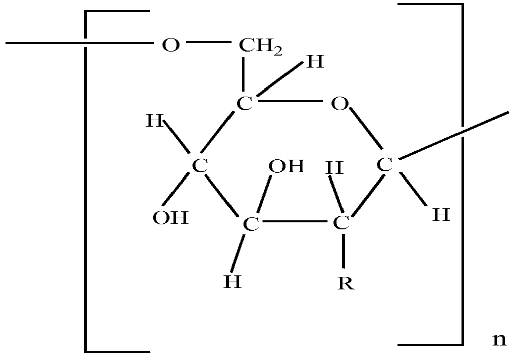 wherein n is 5 or greater, R is selected from the group consisting of -NH-CO-CH₃ and -NH₂, provided that less than 50% of the R groups are -NH-CO-CH₃.

There is also described a pharmaceutical composition comprising an isolated polysaccharide having the formula 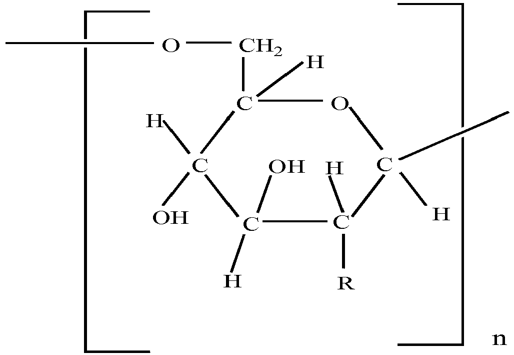 wherein n is at least 5, R is selected from the group consisting of -NH-CO-CH₃ and -NH₂, provided that less than 50% of the R groups are -NH-CO-CH₃, for use in preventing or treating, in a subject, an infection by a non-*ica*/*pga* PNAG-positive pathogen.

There is also described a pharmaceutical composition comprising an isolated polysaccharide conjugated to a carrier, wherein the polysaccharide has the formula 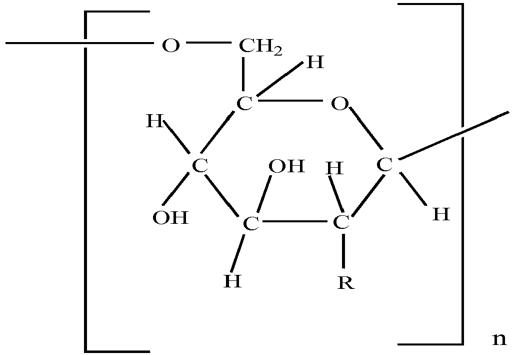 wherein n is 5 or greater, R is selected from the group consisting of -NH-CO-CH₃ and -NH₂, provided that less than 50% of the R groups are -NH-CO-CH₃, for use in preventing or treating, in a subject, an infection by a non-*ica*/*pga* PNAG-positive pathogen.

In some embodiments, the isolated polysaccharide is conjugated to the carrier through a linker. In some embodiments, the carrier is a peptide carrier. Each polysaccharide may be conjugated to one or more carriers. The carrier may be a polysaccharide. In some embodiments, the carrier polysaccharide is not an N-acetyl beta (β) 1-6 glucosamine.

In some embodiments, equal to or less than 45%, equal to or less than 40%, equal to or less than 35%, equal to or less than 30%, equal to or less than 25%, equal to or less than 20%, equal to or less than 15%, equal to or less than 10%, equal to or less than 5%, or equal to or less than 1% of R groups are -NH-CO-CH₃. In some embodiment, none of the R groups is - NH-CO-CH₃.

In some embodiments, n is at least 9, at least 10, at least 20, at least 50, at least 100, at least 200, at least 300, at least 400 or at least 500.

In some embodiments, the isolated polysaccharide has a molecular weight of 100-500 kDa. In some embodiments, the isolated polysaccharide has a molecular weight of at least 900 Daltons, at least 2000 Daltons, at least 2500 Daltons, at least 5000 Daltons, at least 7500 Daltons, at least 10,000 Daltons, at least 25,000 Daltons, at least 50,000 Daltons, at least 75,000 Daltons, at least 100,000 Daltons, at least 125,000 Daltons, at least 150,000 Daltons, at least 200,000 Daltons, at least 250,000 Dalton, at least 300,000 Daltons, at least 350,000 Daltons, at least 400,000 Daltons, at least 450,000 Daltons, or at least 500,000 Daltons.

In some embodiments, the isolated polysaccharide is administered or formulated with an adjuvant or is used in conjunction with an adjuvant.

In some embodiments, the isolated polysaccharide is administered systemically or is formulated for systemic administration. In some embodiments, the isolated polysaccharide is administered locally or is formulated for local administration.

In some embodiments, the isolated polysaccharide is provided in a composition that further comprises a pharmaceutically acceptable carrier.

There is described a method comprising administering to a subject having or at risk of developing an infection by a non-*ica*/*pga* PNAG-positive pathogen an effective amount of a PNAG-specific antibody or PNAG-specific antibody fragment.

There is also described a pharmaceutical composition comprising a PNAG-specific antibody or PNAG-specific antibody fragment for use in preventing or treating, in a subject, an infection by a non-*ica*/*pga* PNAG-positive pathogen.

In some embodiments, the non-*ica*/*pga* PNAG-positive pathogen is a non-*ica*/*pga* PNAG-positive gram-positive coccus. In some embodiments, the non-*ica*/*pga* PNAG-positive gram-positive coccus is *S. pneumonia*, Group A *Streptococcus*, Group B *Streptococcus*, Group C *Streptococcus,* or *Enterococcus.*

In some embodiments, the non-*ica*/*pga* PNAG-positive pathogen is a non-*ica*/*pga* PNAG-positive gram-positive rod. In some embodiments, the non-*ica*/*pga* PNAG-positive gram-positive rod is *Listeria*, *Clostridium difficile*, *B. subtilis*, *M. tuberculosis*, or *M. smegmatis.*

In some embodiments, the non-*ica*/*pga* PNAG-positive pathogen is a non-*ica*/*pga* PNAG-positive gram-negative coccus or coccobacillus. In some embodiments, the non-*ica*/*pga* PNAG-positive gram-negative coccus or coccobacillus is *Neisseria meningitides*, *Neisseria gonorrhoeae*, Non-typable *H. Influenzae*, *Helicobacter spp*, or *Campylobacter spp.*

In some embodiments, the non-*ica*/*pga* PNAG-positive pathogen is a non-*ica*/*pga* PNAG-positive gram-negative rod. In some embodiments, the non-*ica*/*pga* PNAG-positive gram-negative rod is *Bacteroides fragilis, B. thetaiotamicron, B. vulgatis, Citrobacter rodentium*, *Vibrio cholerae*, *Salmonella enterica* serovar typhi and *Salmonella enterica* serovar typhimurium. In some embodiments, the non-*ica*/*pga* PNAG-positive pathogen is a non-*ica*/*pga* PNAG-positive fungus. In some embodiments, the non-*ica*/*pga* PNAG-positive fungus is *Candida albicans* (yeast); *Candida albicans* (hyphae), *Aspergillus*, *Fusarium*, or *Cryptococcus.*

In some embodiments, the non-*ica*/*pga* PNAG-positive pathogen is a non-*ica*/*pga* PNAG-positive parasite. In some embodiments, the non-*ica*/*pga* PNAG-positive parasite is *P. bergei* or *P. falciparum.*

In some embodiments, the non-*ica*/*pga* PNAG-positive pathogen is *T. vaginalis.*

In some embodiments, the subject is human. In some embodiments, the subject is a primate, horse, cow, swine, goat, sheep, dog, or cat.

In some embodiments, the subject has an infection by a non-*ica*/*pga* PNAG-positive pathogen. In some embodiments, the subject is at risk of developing an infection by a non-*ica*/*pga* PNAG-positive pathogen.

In some embodiments, the antibody or antibody fragment is administered systemically or is formulated for systemic administration. In some embodiments, the antibody or antibody fragment is administered locally or is formulated for local administration.

There is described a method comprising ethanol precipitating a crude polysaccharide preparation from a concentrated microbial cell body preparation; concurrently digesting the crude polysaccharide with lysozyme and lysostaphin followed by sequential digestion with a nuclease and proteinase K to form a digested polysaccharide preparation; size fractionating the digested polysaccharide preparation; isolating an acetylated polysaccharide fraction; and de-acetylating the acetylated polysaccharide fraction to produce a PNAG polysaccharide having less than 50% acetate substitutions, wherein the microbial cell body preparation is derived from a non-*ica*/*pga* PNAG-positive microbe. In some embodiments, the polysaccharide preparation is size fractionated using a column. In some embodiments, the method produces PNAG polysaccharide having less than 40% acetate substitutions.

There is also described a method comprising preparing an impure polysaccharide from a microbial culture; incubating the impure polysaccharide with an acid or a base to produce a semi-pure polysaccharide preparation; neutralizing the preparation; incubating the neutralized preparation in hydrofluoric acid; isolating an acetylated polysaccharide from the preparation; and de-acetylating the acetylated polysaccharide to produce a PNAG polysaccharide having less than 50% acetate substitutions, wherein the microbial culture is a non-*ica*/*pga* PNAG-positive microbial culture. In some embodiments, the method produces PNAG polysaccharide having less than 40% acetate substitutions.

There is also described a method comprising preparing an impure polysaccharide from a microbial culture; incubating the impure polysaccharide with an acid or a base to produce a semi-pure polysaccharide preparation; neutralizing the preparation; incubating the neutralized preparation in hydrofluoric acid; and isolating from the preparation a PNAG polysaccharide having less than 50% acetate substitutions, wherein the microbial culture is a non-*ica*/*pga* PNAG-positive microbial culture. In some embodiments, PNAG polysaccharide having less than 40% acetate substitutions is isolated.

In some embodiments, the method further comprises conjugating a carrier to the isolated polysaccharide. In some embodiments, the carrier is a peptide carrier.

In some embodiments, the acetylated polysaccharide is chemically de-acetylated.

In some embodiments, the acetylated polysaccharide is de-acetylated by incubation with a basic solution. In some embodiments, the acetylated polysaccharide is enzymatically de-acetylated.

There is described a method for producing antibodies comprising administering to a subject an effective amount for producing antibodies of an PNAG polysaccharide isolated from a non-*ica*/*pga* PNAG-positive pathogen, and an adjuvant, and isolating antibodies from the subject. In some embodiments, the antibodies are polyclonal antibodies.

There is also described a method for producing monoclonal antibodies comprising administering to a subject an effective amount for producing antibodies of a PNAG polysaccharide isolated from a non-*ica*/*pga* PNAG-positive pathogen, and an adjuvant, harvesting spleen cells from the subject, fusing spleen cells from the subject to myeloma cells, and harvesting antibody produced from a fusion subclone.

In some embodiments, the method further comprises isolating antibody.

In some embodiments, the PNAG polysaccharide is less than 50% acetylated.

In some embodiments, the subject is a rabbit. In some embodiments, the subject is human.

There is also described a method for detecting a non-*ica*/*pga* PNAG-positive pathogen, comprising contacting a sample suspected of containing a non-*ica*/*pga* PNAG-positive pathogen with a PNAG-specific antibody or antibody fragment, and detecting binding of the antibody or antibody fragment to the sample, wherein binding of the antibody or antibody fragment indicates the non-*ica*/*pga* PNAG-positive pathogen is present in the sample.

In some embodiments, the sample is Staphylococcus negative.

In some embodiments, the sample is a biological sample from a subject. In some embodiments, the biological sample is urine, blood, pus, skin, sputum, joint fluid, lymph or milk. In some embodiments, the sample is derived from a swab of an implantable or implanted medical device or a piece of medical equipment or a surface in a patient care facility.

In some embodiments, the antibody or antibody fragment is a humanized antibody or a chimeric antibody or a fragment thereof. In some embodiments, the antibody is a human antibody. In some embodiments, the antibody or antibody fragment is F598 (ATCC PTA-5931) antibody or a fragment thereof. In some embodiments, the antibody or antibody fragment is F628 (ATCC PTA-5932) antibody or a fragment thereof. In some embodiments, the antibody or antibody fragment is F630 (ATCC PTA-5933) antibody or a fragment thereof. Polyclonal antisera raised to PNAG can also be used in some instances.

In some embodiments, the antibody or antibody fragment is conjugated to an agent.

In some embodiments, the agent is a cytotoxic agent such as an antibiotic or a radioisotope. In some embodiments, the agent is a detectable label. In some embodiments, the detectable label is a radioactive label, an enzyme, a biotin molecule, an avidin molecule or a fluorochrome.

Each of the limitations of the invention can encompass various embodiments of the invention. It is therefore anticipated that each of the limitations of the invention involving any one element or combinations of elements can be included in each aspect of the invention.

### BRIEF DESCRIPTION OF THE FIGURES

FIG. 1. Effect of MAb F598 to PNAG given intraperitoneally (ip) and topically starting at 4 hours post-infection. This Figure shows data from the lowest inoculum, 48 hour experiment. Experimental details: Inoculum was 1x10⁵ /eye; 200 µg of MAbs injected ip 4 and 24 hours post-infection; 20 µg MAbs applied topically 24 and 32 hours post-infection. Data points represent value for individual mouse; bars represent mean score for the group. P value: Mann Whitney U test.
FIG. 2. Effect of MAb F598 to PNAG given IP and topically starting at 4 hours post-infection. This Figure shows data from the low inoculum, 48 hour experiment. Experimental details: Inoculum was 2x10⁵/eye; 500 µg of MAbs injected IP 4 hours post-infection; 50 µg of MAbs applied topically 24 and 32 hours post-infection. Data points represent value for individual mouse; bars represent mean score for the group.
   P value: Mann Whitney U test.
FIG. 3. Effect of MAb F598 to PNAG given topically starting 4 hours post-infection. This Figure shows data from the medium inoculum, 32 hour experiment. Experimental details: Inoculum was 5.1x10⁶/eye; MAbs applied topically 4, 8, 24 hours post-infection. Data points represent value for individual mouse; bars represent mean score for the group.
   P value: Mann Whitney U test.
FIG. 4. Effect of MAb F598 to PNAG given topically starting 4 hours post-infection This Figure shows data from the high inoculum, 32 hour experiment. Experimental details:
   Inoculum: 5x10⁷/eye; MAbs applied topically 4, 8, 24 hours post-infection. Experiment was terminated at 32 hours. Data points represent value for individual mouse; bars represent mean score for the group. P value: Mann Whitney U test.
FIG. 5. Survival of CBA/N mice challenged with *S. pneumoniae* D39 (N=12/group).
FIG. 6. Protective efficacy of antibody raised to the 9GlcNH2-TT conjugate vaccine against lethal skin infection cause from *S. pyogenes* (Group A *Streptococcus*).
FIG. 7. Protective efficacy of antibody raised in rabbits to the 9GlcNH2-TT conjugate vaccine against meningitis (bacteria in the brain) of 2-3 day old mouse pups challenged with Group B *N. meningitides* strain B16B6. Data points represent log₁₀ CFU/brain for an individual mouse; bars represent median score for the group. P value: Mann Whitney U test.
FIG. 8. Reductions in colitis scores in mice administered human IgG1 MAb to PNAG compared to either PBS (experiment 1) or a human IgG1 MAb to HIV (MAb F105, experiment 2). Data points represent score for individual mouse; bars represent median score for the group. P value: Mann Whitney U test.
FIG. 9. Comparison of the individual scores for the four parameters used to find the total histologic score in TRUC mice treated with either MAb F598 to PNAG or control human IgG1 MAb to HIV (F105).
FIG. 10. Protective efficacy of antibody raised in rabbits to the 9GlcNH₂-TT conjugate vaccine against *L. monocytogenes.*
FIG. 11. Opsonic killing of 4 strains of *S. pneumoniae* mediated by rabbit antibody raised to dPNAG-TT. Killing compared to that obtained in control with normal rabbit serum.
FIG. 12. Opsonic killing of 4 strains of *S. pneumoniae* mediated by human IgG1 MAb F598 to PNAG20. Killing compared to control MAb F429 specific to *P. aeruginosa* alginate.
FIG. 13. Opsonic killing of 3 strains of *E. faecalis* mediated by rabbit antibody raised to 9GlcNH₂-TT. Killing compared to that obtained in control with normal rabbit serum.
FIG. 14. Opsonic killing of Group A *Streptococcus* by MAb F598 to PNAG. Killing compared to control MAb F429 specific to P. aeruginosa alginate.
FIG. 15. Opsonic killing of *Candida albicans* by MAb F598 to PNAG. Killing compared to control MAb F429 specific to *P. aeruginosa* alginate.
FIG. 16. Bactericidal killing of *N. meningitidis* serogroup B strains.
FIG. 17. Bactericidal killing of *N. gonorrhoeae.*
FIG. 18. Inhibition of *N. meningitidis* bactericidal killing.
FIG. 19. Inhibition of *N. gonorrhoeae* bactericidal killing.

### DETAILED DESCRIPTION OF INVENTION

The invention relates, in part, to the unexpected finding of PNAG expression on a number of bacterial and non-bacterial pathogens. The finding was unexpected for at least two reasons. First, none of the pathogens which were found to express PNAG, in accordance with the invention, have an *ica* or a *pga* locus. *Ica* and *pga* loci each encodes four proteins involved in polysaccharide synthesis, including PNAG synthesis. It had been thought, prior to the invention, that a pathogen must have an *ica* or the *pga* locus in order to synthesize PNAG. The *ica* locus is present in *S. aureus* and *S. epidermidis*, which were known to express PNAG prior to the invention, while the *pga* locus has been identified in some gram-negative organisms. It is not clear how the newly discovered PNAG-positive pathogens actually synthesize PNAG in the apparent absence of these loci. The findings of the invention suggest that PNAG may be synthesized even in the absence of such loci and the proteins they encode. Second, there is great variety in the pathogens founds to express PNAG, including bacterial and non-bacterial pathogens. Prior to the invention, it was not contemplated that pathogens that did not express a discernible *ica*/*pga* locus could make PNAG. It was also not contemplated that non-bacterial pathogens might express PNAG.

The finding that these various bacterial and non-bacterial pathogens express PNAG provides new approaches for preventing, treating and/or diagnosing infections caused by such pathogens. Thus, there is described, inter alia, isolation and/or derivation of PNAG and dPNAG from the newly described PNAG-positive pathogens, and their use in stimulating immune responses (including immune responses required to produce antibodies specific for PNAG), detecting PNAG and PNAG-expressing pathogens, and preventing and treating infections of PNAG-expressing pathogens. Such PNAG-expressing pathogens include but are not limited to the non-*ica*/*pga* PNAG-expressing pathogens described herein.

### Non-ica/pga PNAG-positive pathogens

The pathogen newly discovered to express PNAG are referred to herein as non-*ica*/*pga* PNAG-positive pathogens to indicate that they do not contain a DNA-based genetic locus with any significant similarity to the four-gene *ica*/*pga* loci of known PNAG-expressing pathogens such as *S. aureus*, *S. epidermidis* or *E. coli.* The *ica or pga* loci encode four proteins (2 glycosyltransferases, an N-deacetylase, and a protein for export of the synthesized polysaccharide). Some non-*ica*/*pga* PNAG pathogens do not comprise genes encoding these four proteins in a single locus.

The nucleotide sequence of an exemplary *ica* locus (i.e., one from *S. aureus*) has been deposited in GenBank under accession number AF086783. A pathogen that is considered a "non-*ica*" pathogen, according to the invention, does not possess a discernible *ica* locus. As an example, such a pathogen may not possess a nucleotide sequence occurring in the same contiguous stretch of chromosomal DNA and having at least 25% homology to the entire 4-gene nucleotide sequence of the *ica* locus deposited under AF086783. Non-*ica* PNAG-positive pathogens exclude *Staphylococci.*

The nucleotide sequence of an exemplary *pga* locus (i.e., one from *E. coli* K12 substr. MG1655) has been deposited in GenBank under accession numbers for each of the 4 genes within the locus as AAC74106.1, AAC74107.1, AAC74108.1, and AAC74109.1. A pathogen that is considered a "*non-pga*" pathogen, according to the invention, does not possess a discernible *pga* locus. As an example, such a pathogen may not possess a nucleotide sequence occurring in the same contiguous stretch of chromosomal DNA and having at least 25% homology to all four of the nucleotide sequences deposited under AAC74106.1, AAC74107.1, AAC74108.1 and AAC74109.1. Non-*pga* PNAG-positive pathogens exclude *E. coli*, *Klebsiella pneumoniae, Bordetella pertussis, B. parapertussis, B. bronchoseptica, Burkholderia cenocepacia, B. dolosa, Actinobacillus pleuropneumoniae, Aggregatibacter actinomycetemcomitans, Acinetobacter baumannii*, and some strains of the genus *Shigella.*

The non-*ica*/*pga* PNAG-positive pathogens include gram-negative and gram-positive bacteria, fungi and parasites. More specifically, the non-*ica*/*pga* PNAG-positive bacteria include gram-positive cocci, gram-positive rods, gram-negative cocci or coccobacilli, and gram-negative rods. The non-*ica*/*pga* PNAG-positive gram-positive cocci include *S*. *pneumoniae*, Group A *Streptococcus* (*Streptococcus pyogenes*), Group B *Streptococcus* (*Streptococcus agalactiae*), Group C *Streptococcus* (*Streptococcus dysagalactiae*), and *Enterococcus* (*E. faecalis* and *E. faecium*). The non-*ica*/*pga* PNAG-positive gram-positive rods include *Listeria monocytogenes, Clostridium difficile*, *Bacillus subtilis, Mycobacterium tuberculosis*, and *M*. *smegmatis.* The non-*ica*/*pga* PNAG-positive gram-negative cocci or coccobacilli include *Neisseria meningitides, Neisseria gonorrhoeae*, Non-typable *H. influenzae*, *Hemophilus ducreyi, Helicobacter pylori*, and *Campylobacter jejuni.* The non-*ica*/*pga* PNAG-positive gram-negative rod includes *Bacteroides fragilis, B. thetaiotamicron, B. vulgatis, Citrobacter rodentium*, *Vibrio cholerae, Salmonella enterica* serovar typhi and *Salmonella enterica* serovar typhimurium.

The non-*ica*/*pga* PNAG-positive fungus include *Candida albicans* (yeast), *Candida albicans* (hyphae), *Aspergillus*, *Fusarium*, and *Cryptococcus* species. The non-*ica*/*pga* PNAG-positive parasites include *Plasmodium bergei* and *P. falciparum.*

The non-*ica*/*pga* PNAG-positive pathogen may be *T. vaginalis.*

There is described the use of the PNAG polysaccharide as an antigen to induce immune responses that are specific for the PNAG polysaccharide in subjects. Such immunity is referred to herein as active immunity. The subjects may be those having or at risk of developing infections caused by any one of the foregoing non-*ica*/*pga* PNAG-positive pathogens. The infections may be prevented or treated through the use of the PNAG polysaccharide.

There is also described the use of PNAG-specific antibodies (or antibody fragments) to induce immune responses that are specific for the PNAG polysaccharide in subjects. Such immunity is referred to herein as passive immunity. The subjects may be those having or at risk of developing infections caused by any one of the foregoing non-*ica*/*pga* PNAG-positive pathogens. The infections may be prevented or treated through the use of the PNAG-specific antibodies (or antibody fragments).

### PNAG and dPNAG polysaccharide

The PNAG polysaccharide is poly N-acetyl beta (β) 1-6 glucosamine (i.e., it is comprised of glucosamine monomer units linked together by beta (β) 1-6 linkages). The acetyl group, when present, is N-linked to the glucosamine monomer (as opposed to being O-linked). PNAG has the structure of the following formula 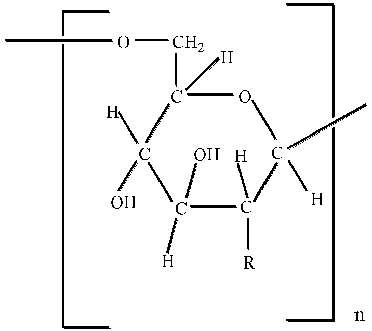 where n is an integer and R is selected from the group consisting of -NH-CO-CH₃ and -NH₂. "n" may range, without limitation, from 2-500.

PNAG may be synthesized in vitro or it may be isolated from a naturally occurring source, such as for example the newly described PNAG-positive pathogens. In its native form, PNAG exists as a mixture of forms ranging in acetylation (i.e., where R is -NH-CO-CH₃) from 1-100%, with the more highly acetylated forms (i.e., those having greater than 50% acetylation) being the more predominant forms.

It was previously discovered that poorly acetylated forms were highly immunogenic and better able to elicit opsonic protective antibodies as compared to the more highly acetylated forms in in vivo immune stimulation assays. The antibodies elicited following dPNAG administration recognize dPNAG and, in some instances, the highly acetylated forms of PNAG also. These findings made the poorly acetylated form of PNAG a suitable vaccine candidate for stimulating protective immune responses in vivo. As a result, the use of the poorly acetylated forms of PNAG to stimulate active immunity in subjects is described. Such poorly acetylated forms of PNAG are referred to herein as deacetylated PNAG (or dPNAG). dPNAG has the same structure as that shown above with the exception that less than 50% of the R groups are -NH-CO-CH₃ (i.e., less than 50% of the amino groups are substituted with acetate). dPNAG may be wholly or partially deacetylated, provided that the range of acetylation is from 0 to less than 50%. Wholly deacetylated dPNAG (i.e., where R=NH₂ only) may be referred to herein as a homopolymer. Partially deacetylated dPNAG (i.e., wherein R may be -NH₂ or -NH-CO-CH₃, provided that less than 50% of R are - NH-CO-CH₃) may be referred to herein as a heteropolymer. For instance, less than 49%, less than 45%, less than 40%, less than 35%, less than 30%, less than 25%, less than 20%, less than 15%, less than 10%, less than 5%, or less than 1% of R groups may be -NH-CO-CH₃. In some instances, the level of acetylation is 40% or less, 35% or less, 20% or less, or 15% or less.

There is described the use of highly acetylated and poorly acetylated forms of PNAG in various applications. As a non-limiting example, highly acetylated PNAG may be used for making antibodies to be used as a diagnostic or for another non-therapeutic purpose.

There is also described the use of naturally occurring forms of PNAG, whether highly or poorly acetylated, as well as synthetic forms of PNAG (i.e., those made completely de novo). As will be appreciated, such synthetic forms can be synthesized with a known number and sequence glucosamine and N-acetyl glucosamine units that are β-1-6 linked to each other. The synthetic forms may be as small as 4 monomers in some instances.

Published U.S. patent application No. US-2011-0150880 describes synthetic oligosaccharides, their synthesis, and their conjugation to carriers. Synthetic oligosaccharides may be used conjugated to a carrier, in some embodiments. An example is an oligosaccharide-carrier conjugate comprising an oligosaccharide conjugated to a carrier through a linker that is 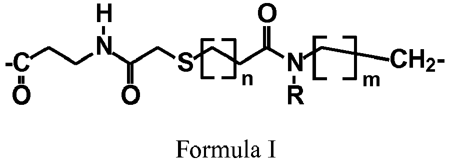 or 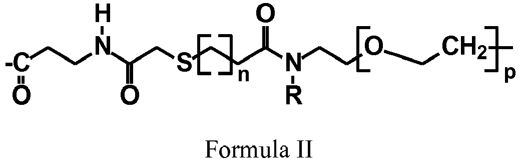 wherein n is greater than 1, m is a number selected from 1 to 10, p is a number selected from 1 to 20, and R is H or an alkyl group, and wherein the linker is O-linked to the oligosaccharide and N-linked to the carrier. "n" may be 2-10, 2-5, or 2, 3, or 4, in some embodiments.

Another example is an oligosaccharide bearing an O-linked linker, wherein the linker comprises 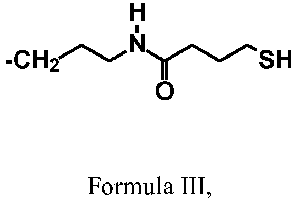 wherein the oligosaccharide is a polyglucosamine. The polyglucosamine may be a β-1-6 linked glucosamine that is 2-20 monomers in length, 5-11 monomers in length, for example.

The size of PNAG and dPNAG may vary and may be dictated by the particular application. Typically PNAG and dPNAG molecular weight may range from about 900 Daltons (Da) to 750 kiloDaltons (kDa). In some aspects, PNAG or dPNAG has a molecular weight of less than 2 kDa. In some embodiments, the molecular weight of PNAG or dPNAG may be at least about 2200 Daltons, or at least about 2500 Daltons, or at least about 3000 Daltons. In some embodiments, PNAG or dPNAG may be at least 9, at least 10 monomer units in length, or at least 12 monomer units in length, or at least 15 monomer units in length. In other aspects, PNAG or dPNAG has a molecular weight of at least 100 kDa, optionally in the range of 100-500 kDa.

As discussed in greater detail herein, PNAG and dPNAG, including lower molecular weight versions of PNAG and dPNAG, may be conjugated to a carrier such as a carrier protein. When conjugated to a carrier, PNAG and dPNAG may be as small as 2-3 monomer units, but preferably are at least 4-6 monomer units in length. Polysaccharides between 800 Da and 1,000 kDa will be typical. PNAG or dPNAG forms of this size may be synthesized de novo as described herein. When used without a carrier compound, the PNAG or dPNAG may be about 100 kDa or greater.

### Preparation of PNAG

There is described the use of naturally occurring and synthetic forms of dPNAG and PNAG, including dPNAG and PNAG isolated or derived from the non-*ica*/*pga* PNAG-expressing pathogens described herein. As used herein, naturally occurring PNAG or dPNAG is one that exists in, and optionally can be isolated or derived from, naturally-occurring sources.

PNAG and dPNAG antigens may be provided and/or used in isolated form. An isolated polysaccharide, such as isolated dPNAG, is one that has been removed and thus separated at least in part from the environment in which it normally exists or in which it has been synthesized. In some instances, an isolated polysaccharide is sufficiently separated from other compounds to be characterized structurally or functionally. For example, an isolated polysaccharide may be "sequenced" in order to determine its chemical composition.

dPNAG can be isolated from native PNAG or it can be derived from more highly acetylated naturally occurring PNAG using the de-acetylation methods described herein. dPNAG that is synthesized in vitro may also be isolated from its synthesis reaction mixture, thereby separating it from reaction substrates, enzymes, co-factors, catalysts, or spurious reaction products.

PNAG and dPNAG can be prepared from any microbial (including bacterial) strain carrying the *ica* locus. These *ica*-carrying strains include those that naturally express the *ica* locus such as but not limited to *S. epidermis* and *S. aureus.* Specific strains include *S*. *epidermis* RP62A (ATCC number 35984), *S. epidermis* RP12 (ATCC number 35983), *S*. *epidermis* M187, *S. aureus* RN4220 (pCN27), and *S. aureus* MN8 mucoid. *Ica*-carrying strains also include those that have been transformed with the genes in the *ica* locus (e.g., *S*. *carnosus* TM300 (pCN27)).

Native PNAG can be prepared by a variety of methods including extracting a crude native PNAG preparation from a microbial culture, including cells and cell free culture supernatants, resulting in the isolation of a high molecular weight native PNAG-enriched material from the crude PNAG preparation, and obtained initially by precipitating an impure PNAG containing the high molecular weight PNAG-enriched material with a solvent such as methanol, ethanol, acetone or any other organic solvent known to one skilled in the art as being capable of causing the precipitation of polysaccharides from aqueous solutions. The steps of extracting the crude native PNAG preparation and isolating and precipitating the impure native PNAG preparation may be performed using methods known in the art and described in published U.S. application No. US-2005-0118198-A1.

This impure PNAG material then may be purified and de-acetylated to produce dPNAG. De-acetylation may be carried out chemically or enzymatically. Chemical deacetylation, in some instances, may involve incubating impure PNAG preparation with a base or acid to produce a semi-pure PNAG preparation, neutralizing the preparation, and further treating the neutralized preparation to produce dPNAG.

Enzymatic deacetylation typically involves incubating impure PNAG with enzymes, such as bacterial enzymes, that digest biological materials, including cell-wall disrupting agents such as lysozyme, lysostaphin, and proteinase K, and nuclease enzymes such as DNase and RNase to digest DNA and RNA. This is followed by an addition of a solvent that will precipitate PNAG out of solution, collection of the precipitate and re-dissolution of PNAG in a base, such as NaOH or an acid such as HCl, followed by neutralization. The neutralization can be accomplished using a base if the incubation step was performed with an acid, or with an acid if the incubation step was performed with a base. The insoluble fraction from the neutral material is then treated, e.g., by incubation in hydrofluoric acid to produce a pure native PNAG antigen or by re-dissolution in buffers with a pH < 4.0 followed by molecular sieve and/or ionexchange chromatography.

Another isolation method includes the steps of extracting a crude PNAG suspension from a microbial (including bacterial) culture by incubating the culture with a strong base or acid. Preferably, the culture is stirred in the strong base or acid for at least 2 hours, and more preferably at least 5, 10, 15, 18 or 24 hours. The strong base or acid can be any type of strong base or acid, but preferably has a strength of at least 1 M NaOH or HCl. In some embodiments, the strong base or acid is 5 M NaOH or 5 M HCl. The acid or base solution is then subjected to centrifugation to collect the cell bodies. In some embodiments, the extraction procedure is repeated several times. The resultant acid or base solution is neutralized to approximately pH 7 and then dialyzed to produce insoluble impure PNAG.

dPNAG can also be synthesized *de novo.* Methods for de novo synthesis of dPNAG are described in published U.S. patent application Nos. US-2005-0118198-A1 and US-2011-0150880-A1.

Some methods may derive dPNAG from starting materials such as but not limited to polyglucose (i.e., dextran), polyglucosamines such as chitin or chitosan, polyglucosaminouronic acid, and polygalactosaminouronic acid may also be used to produce the dPNAG antigen of the invention.

PNAG and dPNAG preparations may be of varying purity. As used herein, a pure PNAG or dPNAG preparation is a PNAG or dPNAG preparation that is greater than 92% free of contaminants. These contaminants include galactose, phosphate, teichoic acid, and the like. In some embodiments, PNAG and dPNAG compositions are at least 93%, 94%, 95%, 96%, 97%, 98%, 99% free of contaminants or are 100% free of contaminants. In some embodiments, a dPNAG composition is free of highly acetylated PNAG.

The degree of purity of a PNAG or a dPNAG composition can be assessed by any means known in the art. For example, the purity can be assessed by chemical analysis assays as well as gas chromatography and nuclear magnetic resonance to verify structural aspects of the material.

### Carriers

PNAG and dPNAG, whether synthesized de novo or derived from a naturally occurring source, may be used in a conjugated or an unconjugated form. In a conjugated form, PNAG or dPNAG may be conjugated to a carrier (or a carrier compound, as the terms are used interchangeably herein), either directly or via a linker. The conjugation can occur at any position in the polysaccharide, including at one or both of its ends.

A "carrier" as used herein is a compound that can be conjugated to a polysaccharide either directly or through the use of a linker. The carrier may be immunologically active (i.e., immunogenic) or it may be inert. When used in vivo, it should be understood that the carrier is safe for administration to a subject.

Carriers include but are not limited to proteins, or peptides, polysaccharides, nucleic acids, or other polymers, lipids, and small molecules. Carrier proteins include for example, plasma proteins such as serum albumin, immunoglobulins, apolipoproteins and transferrin; bacterial polypeptides such as TRPLE, β- galactosidase, polypeptides such as herpes gD protein, allergens, diphtheria and tetanus toxoids, salmonella flagellin, hemophilus pilin, hemophilus 15 kDa, 28-30kDa and 40 kDa membrane proteins, *Escherichia coli*, heat label enterotoxin ltb, cholera toxin, and viral proteins including rotavirus VP and respiratory syncytial virus f and g proteins.

Carrier proteins that may be particularly useful for immunization include keyhole limpet hemocyanin, serum albumin, bovine thyroglobulin, or soy bean trypsin inhibitor. Any other compound that is immunogenic in the subject being immunized can be used as a carrier.

Many methods are known in the art for conjugating a polysaccharide to a protein. In general, the polysaccharide should be activated or otherwise rendered amenable to conjugation (i.e., at least one moiety must be rendered capable of covalently bonding to a protein or other molecule). Many such methods are known in the art. Reference can be made to published U.S. patent application Nos. US-2005-0118198-A1 and US-2011-0150880-A1 and U.S. Patent Nos. 4356170, 4663160, 4619828, 4808700, 4711779.

The carrier may be conjugated to PNAG or dPNAG through a linker or spacer. A polysaccharide may be coupled to a linker or a spacer by any means known in the art including, for example using a free reducing end of the polysaccharide to produce a covalent bond with a spacer or linker. A covalent bond may be produced by converting a free reducing end of PNAG or dPNAG into a free 1-aminoglycocide, that can subsequently be covalently linked to a spacer by acylation. (Lundquist et al., J. Carbohydrate Chem., 10:377 (1991)). Alternatively, PNAG or dPNAG may be covalently linked to the spacer using an N-hydroxysuccinimide active ester as activated group on the spacer. (Kochetkow, Carbohydrate Research, 146:C1 (1986)). The free reducing end of PNAG or dPNAG may also be converted to a lactone using iodine and potassium hydroxide. (Isebell et al., Methods of Carbohydrate Chemistry, Academic Press, New York (1962)). The lactone can be covalently linked to the spacer by means of a primary amino group on the spacer or linker. The free reducing end of PNAG or dPNAG may also be covalently linked to the linker or spacer using reductive amination.

### Antibodies

The invention embraces antibodies that bind to PNAG and/or dPNAG. The antibodies may be either monoclonal antibodies or polyclonal antibodies. Antibodies that bind to dPNAG may also bind to forms of highly acetylated forms of PNAG. Antibodies may be made using dPNAG or PNAG or synthetic oligosaccharides composed of >3 monosaccharide units of glucosamine or N-acetyl glucosamine, optionally conjugated to a carrier and/or used in conjunction with an adjuvant. Antibodies may be produced using PNAG or dPNAG derived from the non-*ica*/*pga* PNAG-positive pathogens or *ica*-carrying or *pga*-carrying pathogens.

Polyclonal antibodies generally are raised in animals by multiple subcutaneous or intraperitoneal injections of an antigen and an adjuvant. Polyclonal antibodies to PNAG or dPNAG or conjugated synthetic oligosaccharides can be generated by injecting PNAG or dPNAG in conjugated or unconjugated form or the synthetic oligosaccharides in a conjugated form, alone or in combination with an adjuvant. Methods for making such polyclonals is described in published U.S. patent application No. US-2005-0118198-A1.

Briefly, dPNAG or dPNAG, in conjugated or unconjugated form, or conjugated oligosaccharides, are combined with an adjuvant such as Freund's incomplete adjuvant (e.g., 100 µg of conjugate for rabbits or mice in 1-3 volumes of Freund's) and injected intradermally at multiple sites. Approximately one month later, the animals are boosted with 1/5 - 1/10 of the original amount of antigen, or antigen conjugate, in adjuvant by subcutaneous injection at multiple sites. One to two weeks later the animals are bled, and the serum is assayed for the presence of antibody. The animals may be repeatedly boosted until the antibody titer plateaus. The animal may be boosted with PNAG or dPNAG or synthetic oligosaccharide conjugates alone, PNAG or dPNAG conjugate or synthetic oligosaccharide conjugates, or PNAG or dPNAG conjugated to a different carrier compound, or synthetic oligosaccharide conjugates, with or without an adjuvant. In some embodiments, the boosts may comprise PNAG rather than dPNAG, or they may contain a mixture of dPNAG and PNAG.

In addition to supplying a source of polyclonal antibodies, the immunized animals can be used to generate PNAG-specific and dPNAG-specific monoclonal antibodies. As used herein, the term "monoclonal antibody" refers to a homogenous (i.e., single clonal) population of immunoglobulins that bind to the same epitope of an antigen. Monoclonal antibodies have the same Ig gene rearrangement and thus demonstrate identical binding specificity. In the case where dPNAG or synthetic oligosaccharide conjugates is used to generate the antibodies, the epitope may be present in highly acetylated PNAG as well as dPNAG and thus antibodies raised against dPNAG may also bind to PNAG.

Methods for preparing monoclonal antibodies are known in the art. Monoclonal antibodies can be prepared by a variety of methods. In one such method, spleen cells isolated from the immunized animal are immortalized by fusion with myeloma cells or by Epstein Barr Virus transformation, and clones expressing the desired antibody are screened and identified. Other methods involve isolation of rearranged Ig gene sequences and cloning into immortalized cell lines. Such methods are described in greater detail in published U.S. patent application Nos. US-2005-0118198-A1 and US-2011-0150880-A1.

Antibodies specific for PNAG may be, without limitation, murine, human or chimeric antibodies such as but not limited to humanized antibodies.

Human monoclonal antibodies may be made by any of the methods known in the art, including those disclosed in U.S. Patent No. 5567610, U.S. Patent No. 5565354, U.S. Patent No. 5571893, Kozber, J. Immunol. 133: 3001 (1984), Brodeur, et al., Monoclonal Antibody Production Techniques and Applications, p. 51-63 (Marcel Dekker, Inc, new York, 1987), and Boerner et al., J. Immunol., 147: 86-95 (1991). Human antibodies may be obtained by recovering antibody-producing lymphocytes from the blood or other tissues of humans producing antibody to an antigen of interest (e.g., dPNAG or PNAG). These lymphocytes can be treated to produce cells that grow on their own in the laboratory under appropriate culture conditions. The cell cultures can be screened for production of antibody to the antigen of interest and then cloned. Clonal cultures can be used to produce human monoclonal antibodies to dPNAG or PNAG, or the genetic elements encoding the variable portions of the heavy and light chain of the antibody can be cloned and inserted into nucleic acid vectors for production of antibody of different types. In addition to the conventional methods for preparing human monoclonal antibodies, such antibodies may also be prepared by immunizing transgenic animals that are capable of producing human antibodies (e.g., Jakobovits et al., PNAS USA, 90: 2551 (1993), Jakobovits et al., Nature, 362: 255-258 (1993), Bruggermann et al., Year in Immunol., 7:33 (1993) and U.S. Patent No. 5569825 issued to Lonberg).

As used herein, a "humanized monoclonal antibody" is a monoclonal antibody or functionally active fragment thereof having at least human constant regions and an antigen-binding region, such as one, two or three CDRs, from a non-human species. Humanized antibodies have particular clinical utility in that they specifically recognize antigens of interest, but will not evoke an immune response in humans against the antibody itself. As an example, murine CDRs may grafted into the framework region of a human antibody to prepare the humanized antibody. See, e.g., L. Riechmann et al., Nature 332, 323 (1988); M. S. Neuberger et al., Nature 314, 268 (1985) and EPA 0 239 400. Alternatively, humanized monoclonal antibodies may be constructed by replacing the non-CDR regions of a non-human antibody with similar regions of human antibodies while retaining the epitopic specificity of the original antibody. For example, non-human CDRs and optionally some of the framework regions may be covalently joined to human FR and/or Fc/pFc' regions to produce a functional antibody. There are commercial entities in the United States that will synthesize humanized antibodies from specific murine antibody regions, such as Protein Design Labs (Mountain View California), Abgenix, and Medarex. Reference may also be made to EP Patent Application No. 0239400.

Antigen-binding antibody fragments are also encompassed by the invention. As is known in the art, only a small portion of an antibody molecule, the paratope, is involved in the binding of the antibody to its epitope (see, in general, Clark, W.R. (1986) The Experimental Foundations of Modern Immunology Wiley & Sons, Inc., New York; Roitt, I. (1991) Essential Immunology, 7th Ed., Blackwell Scientific Publications, Oxford). The pFc' and Fc regions of the antibody, for example, are effectors of the complement cascade but are not involved in antigen binding. An antibody from which the pFc' region has been enzymatically cleaved, or which has been produced without the pFc' region, designated an F(ab')₂ fragment, retains both of the antigen binding sites of an intact antibody. An isolated F(ab')₂ fragment is referred to as a bivalent monoclonal fragment because of its two antigen binding sites. Similarly, an antibody from which the Fc region has been enzymatically cleaved, or which has been produced without the Fc region, designated an Fab fragment, retains one of the antigen binding sites of an intact antibody molecule. Proceeding further, Fab fragments consist of a covalently bound antibody light chain and a portion of the antibody heavy chain denoted Fd (heavy chain variable region). The Fd fragments are the major determinant of antibody specificity (a single Fd fragment may be associated with up to ten different light chains without altering antibody specificity) and Fd fragments retain epitope-binding ability in isolation.

The terms Fab, Fc, pFc', F(ab')₂ and Fv are employed with either standard immunological meanings [Klein, Immunology (John Wiley, New York, NY, 1982); Clark, W.R. (1986) The Experimental Foundations of Modern Immunology (Wiley & Sons, Inc., New York); Roitt, I. (1991) Essential Immunology, 7th Ed., (Blackwell Scientific Publications, Oxford)]. Well-known functionally active antibody fragments include but are not limited to F(ab')₂, Fab, Fv and Fd fragments of antibodies. These fragments which lack the Fc fragment of intact antibody, clear more rapidly from the circulation, and may have less non-specific tissue binding than an intact antibody (Wahl et al., J. Nucl. Med. 24:316-325 (1983)). For example, single-chain antibodies can be constructed in accordance with the methods described in U.S. Patent No. 4,946,778 to Ladner et al. Such single-chain antibodies include the variable regions of the light and heavy chains joined by a flexible linker moiety. Methods for obtaining a single domain antibody ("Fd") which comprises an isolated variable heavy chain single domain, also have been reported (see, for example, Ward et al., Nature 341:644-646 (1989), disclosing a method of screening to identify an antibody heavy chain variable region (V_{H} single domain antibody) with sufficient affinity for its target epitope to bind thereto in isolated form). Methods for making recombinant Fv fragments based on known antibody heavy chain and light chain variable region sequences are known in the art and have been described, e.g., Moore et al., US Patent No. 4,462,334. Other references describing the use and generation of antibody fragments include e.g., Fab fragments (Tijssen, Practice and Theory of Enzyme Immunoassays (Elsevieer, Amsterdam, 1985)), Fv fragments (Hochman et al., Biochemistry 12: 1130 (1973); Sharon et al., Biochemistry 15: 1591 (1976); Ehrilch et al., U.S. Patent No. 4,355,023) and portions of antibody molecules (Audilore-Hargreaves, U.S. patent No. 4,470,925). Thus, those skilled in the art may construct antibody fragments from various portions of intact antibodies without destroying the specificity of the antibodies for the dPNAG epitope. It is to be understood that the epitope recognized by anti-dPNAG antibodies may also be present on highly acetylated PNAG.

### Uses

The polysaccharides, synthetic oligosaccharides and antibodies of the invention are useful in a variety of different applications including *in vitro*, *in situ* and *in vivo* applications. The polysaccharides and synthetic oligosaccharides may be used to immunize subjects *in vivo* to prevent or treat infection by non-*ica*/*pga* PNAG-positive pathogens. The polysaccharides and synthetic oligosaccharides may also be used to develop PNAG- or dPNAG-specific antibodies which, in turn, may be used to immunize subjects in vivo to prevent or treat infection by non-*ica*/*pga* PNAG-positive pathogens.

PNAG and/or dPNAG derived from non-*ica*/*pga* PNAG-positive pathogens may be used to screen for binding partners such as antibodies. The antibodies may also be used to detect PNAG-expressing pathogens, including detecting (i.e., diagnosing) infection in a subject. There are described methods for generating antibodies that bind to PNAG and dPNAG.

PNAG and/or dPNAG derived from non-*ica*/*pga* PNAG-positive pathogens may be used to induce an immune response in a subject having or at risk of developing an infection by any PNAG-expressing pathogen, including those that carry an *ica* locus and those that do not. It is to be understood that "PNAG and/or dPNAG derived from non-*ica*/*pga* PNAG-positive pathogens" means the polysaccharides produced from non-*ica*/*pga* PNAG-positive pathogens using the methods described herein. Immune response induction may prevent or it may partially or wholly treat the infection. Partial treatment of the infection may include reduction in the severity or frequency of symptoms and/or partial reduction in pathogen load in the subject. Partial treatment may be useful where a subject is being administered or will be administered one or more other therapeutic agents. Immune response induction is accomplished by administering to the subject an effective amount for inducing an immune response such as an antibody response against PNAG or dPNAG (or pathogens expressing PNAG) of any of PNAG or dPNAG or compositions thereof.

As used herein, a subject is a warm-blooded mammal and includes, for instance, humans, primates, horses, cows, swine, goats, sheep, dogs, and cats. In some embodiments, the subject is a non-rodent subject. A non-rodent subject is any subject as defined above, but specifically excluding rodents such as mice, rats, and rabbits. In some embodiments, the preferred subject is a human.

The subject may be one having or one at risk of developing an infection by a PNAG-expressing pathogen whether such pathogen carries an *ica*-locus or not. A subject at risk of developing an infection by a PNAG-expressing pathogen may be at risk of being exposed to such a pathogen. As described herein, a number of the non-*ica*/*pga* PNAG-positive pathogens are resistant to one or more antibiotic classes. It is therefore likely that exposure to such pathogens may occur since they will not have been eradicated in a prior subject through the use of antibiotics. Populations at risk of developing infection include, for example, neonatal subjects, immunocompromised subjects (such as those receiving chemotherapy), subjects using immunosuppressants (including transplant recipients), subjects on dialysis, subjects undergoing high risk surgery, and subjects with indwelling medical devices such as intravenous lines (e.g., central lines) or prostheses (e.g., hip or knee replacement prostheses).

PNAG or dPNAG of and synthetic oligosaccharides conjugated to protein carriers can be administered to the subject in an effective amount for inducing an immune response. Such an effective amount may be an amount sufficient to assist the subject in producing its own immune protection by for example inducing the production of antibodies specific to PNAG and/or dPNAG, inducing the production of memory cells, and possibly a cytotoxic lymphocyte reaction, etc. The immune response may in turn prevent infection by a PNAG-expressing pathogen from occurring in a subject that is exposed to such a pathogen. One of ordinary skill can assess whether an amount of PNAG or dPNAG or synthetic oligosaccharide conjugate vaccines are sufficient to induce active immunity by methods known in the art. For instance, the ability of a PNAG or dPNAG or synthetic oligosaccharide conjugate vaccines to produce PNAG-specific antibody in a mammal can be assessed by screening the produced antibodies in a mouse or other subject using the PNAG antigen. Amounts of PNAG or dPNAG for inducing immune responses may range from about 1 to 100 µg, although they are not so limited.

The antibody or antibody fragment specific for PNAG and/or dPNAG is useful for inducing passive immunization in a subject, for example, by preventing the development of systemic infection in those subjects at risk of exposure to PNAG-expressing pathogens, including non-*ica*/*pga* PNAG-positive pathogens. The method for inducing passive immunity to infection involves administering to a subject an effective amount of an antibody specific for PNAG and/or dPNAG or the synthetic oligosaccharides for inducing an immune response to PNAG or PNAG-expressing pathogens, including non-*ica*/*pga* PNAG-positive pathogens.

The antibody or antibody fragment may be administered to any subject at risk of developing an infection by non-*ica*/*pga* PNAG-positive pathogens, and in some embodiments may be particularly suited for subjects incapable of inducing active immunity to PNAG and/or dPNAG. PNAG or dPNAG or synthetic oligosaccharide conjugate vaccines might not be completely effective at preventing or eliminating an infection in certain subjects, and therefore such subjects may benefit from treatment with antibody specific for PNAG and/or dPNAG. A subject that is incapable of inducing an immune response includes an immunocompromised subject (e.g., a subject undergoing chemotherapy, a subject having AIDS, etc.) or a subject that has not yet developed an immune system (e.g. pre-term neonate).

The antibody or antibody fragment is administered to the subject in an effective amount for inducing an immune response to PNAG or PNAG-expressing pathogens such as non-*ica*/*pga* PNAG-positive pathogens. As used herein, an effective amount or antibody or antibody fragment for inducing an immune response is an amount of antibody or antibody fragment that is sufficient to (i) prevent infection by from occurring in a subject that is exposed to the pathogen; (ii) inhibit the development of infection, i.e., arresting or slowing its development; and/or (iii) relieve the infection, i.e., eradication of the microbe in infected subjects. Microbes include bacteria, viruses, fungi, parasites and the like.

Using procedures known to those of ordinary skill, one can determine whether an amount of antibody or antibody fragment is an effective amount in an *in vitro* opsonization assay which is predictive of the degree of opsonization of an antibody. An antibody that opsonizes a microbe such as a bacterium is one that when added to a sample of microbes causes phagocytosis of the microbes. An opsonization assay may be a colorimetric assay, a chemiluminescent assay, a fluorescent or radiolabel uptake assay, a cell mediated cytotoxic assay or other assay which measures the opsonic potential of a material.

### Pharmaceutical compositions and formulations

In general, when administered in vivo, the polysaccharides, antibodies and antibody fragments of the invention are applied in pharmaceutically acceptable compositions. Such compositions may comprise pharmaceutically acceptable carriers, salts, buffering agents, preservatives, adjuvants, and optionally other prophylactic or therapeutic ingredients. A pharmaceutically-acceptable carrier means one or more compatible solid or liquid filler, diluents or encapsulating substances which are suitable for administration to a human or other animal. In the context of a pharmaceutically acceptable carrier, the term "carrier" denotes an organic or inorganic ingredient, natural or synthetic, with which the polysaccharide, antibody or antibody fragment is combined to facilitate use including administration. The components of the pharmaceutical compositions should also be capable of being commingled with the polysaccharide, antibody or antibody fragment, and with each other, in a manner such that there is no interaction which would substantially impair the desired pharmaceutical efficiency.

Pharmaceutically acceptable salts include, but are not limited to, those prepared from the following acids: hydrochloric, hydrobromic, sulphuric, nitric, phosphoric, maleic, acetic, salicyclic, p-toluene sulphonic, tartaric, citric, methane sulphonic, formic, malonic, succinic, naphthalene-2-sulphonic, and benzene sulphonic. Also, pharmaceutically acceptable salts can be prepared as alkaline metal or alkaline earth salts, such as sodium, potassium or calcium salts of the carboxylic acid group.

Suitable buffering agents include acetic acid and a salt (1-2% W/V); citric acid and a salt (1-3% W/V); boric acid and a salt (0.5-2.5% W/V); and phosphoric acid and a salt (0.8-2% W/V). Suitable preservatives include benzalkonium chloride (0.003-0.03% W/V); chlorobutanol (0.3-0.9% W/V); parabens (0.01-0.25% W/V) and thimerosal (0.004-0.02% W/V).

Compositions suitable for parenteral administration typically comprise a sterile aqueous preparation of the polysaccharide, antibody or antibody fragment, which may be isotonic with the blood of the recipient subject. Among the acceptable vehicles and solvents that may be employed are water, Ringer's solution, and isotonic sodium chloride solution. In addition, sterile, fixed oils are conventionally employed as a solvent or suspending medium. For this purpose any bland fixed oil may be employed including synthetic mono or di-glycerides. In addition, fatty acids such as oleic acid find use in the preparation of injectables. Carrier formulations suitable for subcutaneous, intramuscular, intraperitoneal, intravenous, etc. administrations may be found in Remington's Pharmaceutical Sciences, Mack Publishing Company, Easton, PA.

The polysaccharides, oligosaccharides, antibodies and antibody fragments are administered in effective amounts. Polysaccharide or oligosaccharide doses ranging from 1-100 µg may be effective, depending on the mode of administration. Antibody or antibody fragment doses ranging from 0.1-100 mg/kg and 0.1-20 mg/kg, depending upon the mode of administration, may be effective. The absolute amount will depend upon a variety of factors including whether the administration is performed on a high risk subject not yet infected with the microbes or on a subject already having an infection, the concurrent treatment, the number of doses and the individual patient parameters including age, physical condition, size and weight. These are factors well known to those of ordinary skill in the art and can be addressed with no more than routine experimentation. It is preferred generally that a maximum dose be used, that is, the highest safe dose according to sound medical judgment.

Multiple doses of the polysaccharides, antibodies and/or antibody fragments are contemplated. Generally immunization schemes involve the administration of a high dose of an antigen followed by subsequent lower doses of antigen after a waiting period of several weeks. Further doses may be administered as well. The dosage schedule for passive immunization would be quite different with more frequent administration if necessary. Any regimen that results in an enhanced immune response to microbial infection and/or subsequent protection from infection may be used. Desired time intervals for delivery of multiple doses of a particular antigen can be determined by one of ordinary skill in the art employing no more than routine experimentation. Vaccine doses may be administered over a period of 1 to 6 months, optionally with doses equally spaced apart in time. For antibodies and antibody fragments, dosing intervals generally range from 14-180 days.

A variety of administration routes are available. The particular mode selected will depend upon, for example, the particular condition being treated and the dosage required for therapeutic efficacy. The methods described, generally speaking, may be practiced using any mode of administration that is medically acceptable, meaning any mode that produces effective levels of an immune response without causing clinically unacceptable adverse effects. Preferred modes of administration are parenteral routes. The term "parenteral" includes subcutaneous, intravenous, intramuscular, intraperitoneal, and intrasternal injection, or infusion techniques. Other routes include but are not limited to oral, nasal, dermal, sublingual, and local.

Other delivery systems can include time-release, delayed release or sustained release delivery systems. Such systems can avoid repeated administrations of the polysaccharides of the invention, increasing convenience to the subject and the physician. Many types of release delivery systems are available and known to those of ordinary skill in the art. They include polymer based systems such as polylactic and polyglycolic acid, polyanhydrides and polycaprolactone; nonpolymer systems that are lipids including sterols such as cholesterol, cholesterol esters and fatty acids or neutral fats such as mono-, di and triglycerides; hydrogel release systems; silastic systems; peptide based systems; wax coatings, compressed tablets using conventional binders and excipients, partially fused implants and the like. Specific examples include, but are not limited to: (a) erosional systems in which the polysaccharide is contained in a form within a matrix, found in U.S. Patent Nos. 4,452,775 (Kent); 4,667,014 (Nestor et al.); and 4,748,034 and 5,239,660 (Leonard) and (b) diffusional systems in which an active component permeates at a controlled rate through a polymer, found in U.S. Patent Nos. 3,832,253 (Higuchi et al.) and 3,854,480 (Zaffaroni). In addition, a pump-based hardware delivery system can be used, some of which are adapted for implantation.

### Secondary agents

dPNAG and/or PNAG-specific antibodies may be delivered in conjunction with other agents. The nature of the other agent(s) may depend upon whether the dPNAG or the PNAG-specific antibody is being administered.

For example, when administered to induce active immunity and/or to produce antibody, dPNAG may be used in conjunction with an adjuvant. As used herein, the term adjuvant refers to a substance that is administered in conjunction with (including at the same time, in the same formulation, etc.) an antigen (such as dPNAG) in order to potentiate an antigen-specific immune response. Adjuvants include but are not limited to aluminum compounds, e.g., gels, aluminum hydroxide and aluminum phosphate, and Freund's complete or incomplete adjuvant (e.g., in which the dPNAG antigen is incorporated in the aqueous phase of a stabilized water in paraffin oil emulsion). The paraffin oil may be replaced with different types of oils, e.g., squalene or peanut oil. Other materials with adjuvant properties include BCG (attenuated *Mycobacterium tuberculosis*), calcium phosphate, levamisole, isoprinosine, polyanions (e.g., poly A:U), lentinan, pertussis toxin, lipid A, saponins, QS-21 and peptides, e.g. muramyl dipeptide. Rare earth salts, e.g., lanthanum and cerium, may also be used as adjuvants. The amount of adjuvants depends on the subject and the particular dPNAG antigen used (e.g., the level of acetate substitution) and can be readily determined by one skilled in the art without undue experimentation.

The agent may be an anti-microbial such as an anti-bacterial, an anti-viral, an anti-parasite, an anti-fungal, and the like.

The agent may be an anti-bacterial drug (e.g., an antibiotic), another bacterial antigen, or another anti-bacterial antibody, or mixtures or combinations thereof. The use of antibiotics in the treatment of bacterial infection is routine. The use of antigens for inducing active immunization and antibodies to induce passive immunization is also routine. In this embodiment, a common administration vehicle (e.g., tablet, implant, injectable solution, etc.) could contain both the active agent of the invention and an antibiotic and/or other antigen and/or other antibody. Alternatively, the antibiotic and/or other antigen and/or other antibody can be administered separately. The antibiotic may be conjugated to dPNAG or to an anti-dPNAG antibody.

Anti-bacterial antibiotic drugs are well known and include, without limitation, penicillin G, penicillin V, ampicillin, amoxicillin, bacampicillin, cyclacillin, epicillin, hetacillin, pivampicillin, methicillin, nafcillin, oxacillin, cloxacillin, dicloxacillin, flucloxacillin, carbenicillin, ticarcillin, avlocillin, mezlocillin, piperacillin, amdinocillin, cephalexin, cephradine, cefadoxil, cefaclor, cefazolin, cefuroxime axetil, cefamandole, cefonicid, cefoxitin, cefotaxime, ceftizoxime, cefmenoxine, ceftriaxone, moxalactam, cefotetan, cefoperazone, ceftazidme, imipenem, clavulanate, timentin, sulbactam, neomycin, erythromycin, metronidazole, chloramphenicol, clindamycin, lincomycin, vancomycin, trimethoprim-sulfamethoxazole, aminoglycosides, quinolones, tetracyclines and rifampin. (See Goodman and Gilman's, Pharmacological Basics of Therapeutics, 8th Ed., 1993, McGraw Hill Inc.)

Polysaccharide antigens (other than PNAG and dPNAG) and polysaccharide-specific antibodies (other than PNAG-specific antibodies) are known in the art. Examples include *Salmonella typhi* capsule Vi antigen (Szu, S.C., X. Li, A.L. Stone and J.B. Robbins, Relation between structure and immunologic properties of the Vi capsular polysaccharide, Infection and Immunity. 59:4555-4561 (1991)); *E. Coli* K5 capsule (Vann, W., M.A. Schmidt, B. Jann and K. Jann, The structure of the capsular polysaccharide (K5 antigen) of urinary tract infective Escherichia coli, 010:K5:H4. A polymer similar to desulfo-heparin, European Journal of Biochemistry. 116: 359-364, (1981)); *Staphylococcus aureus* type 5 capsule (Fournier, J.-M., K. Hannon, M. Moreau, W.W. Karakawa and W.F. Vann, Isolation of type 5 capsular polysaccharide from Staphylococcus aureus, Ann. Inst. Pasteur/Microbiol. (Paris). 138: 561-567, (1987)); *Rhizobium melilori* expolysaccharide II (Glazebrook, J. and G.C. Walker, a novel expolysaccharide can function in place of the calcofluor-binding exopolysaccharide in nodulation of alfalfa by Rhizobium meliloti, Cell. 65:661-672 (1989)); Group B *Streptococcus* type III (Wessels, M.R., V. Pozsgay, D.L. Kasper and H. J. Jennings, Structure and immunochemistry of an oligosaccharide repeating unit of the capsular polysaccharide of type III Group B Streptococcus, Journal of Biological Chemistry. 262:8262-8267 (1987)); *Pseudomonas aeruginosa* Fisher 7 O-specific side-chain (Knirel, Y.A., N.A. Paramonov, E.V. Vinogradov, A.S. Shashkow, B.A. N.K. Kochetkov, E.S. Stanislavsky and E.V. Kholodkova, Somatic antigens of Pseudomonas aeruginosa The structure of O-specific polysaccharide chains of lipopolysaccharides of P. aeruginosa O3 (Lanyi), 025 (Wokatsch) and Fisher immunotypes 3 and 7, European Journal of Biochemistry. 167:549, (1987)); *Shigella sonnei* O-specific side chain (Kenne, L., B. Lindberg and K. Petersson, Structural studies of the O-specific side-chains of the Shigella sonnei phase I lipopolysaccharide, Carbohydrate Research. 78:119-126, (1980)); *S. pneumoniae* type I capsule (Lindberg, B., Lindqvist, B., Lonngren, J., Powell, D.A., Structural studies of the capsular polysaccharide from S. pneumoniae type 1, Carbohydrate Research. 78:111-117 (1980)); and *S. pneumoniae* group antigen (Jennings, H.J., C. Lugowski and N. M. Young, Structure of the complex polysaccharide C-substance from S. pneumoniae type 1, Biochemistry. 19:4712-4719 (1980)). Other non-polypeptide antigens and non-polysaccharide specific antibodies are known to the those of skill in the art and can be used in conjunction with the compositions of the invention.

### Detection and diagnostic assays

The dPNAG and synthetic oligosaccharide antigens and antibodies to them are also useful in diagnostic assays for determining an immunologic status of a subject or sample or can be used as reagents in immunoassays. For instance, the antibodies may be used to detect the presence in a sample of a microbe such as a bacterium having PNAG on the surface. If the microbe is present in the sample, then the antibodies may be used to treat the infected subject. The antibodies may also be used to screen microbes for the presence of PNAG antigen and to isolate dPNAG or PNAG antigen and microbes containing dPNAG or PNAG antigen from complex mixtures.

The above-described assays and any other assay known in the art can be accomplished by labeling the dPNAG or antibodies and/or immobilizing the dPNAG or antibodies on an insoluble matrix. The analytical and diagnostic methods for using dPNAG and/or its antibodies use at least one of the following reagents: labeled analyte analogue, immobilized analyte analogue, labeled binding partner, immobilized binding partner, and steric conjugates. The label used can be any detectable functionality that does not interfere with the binding of analyte and its binding partner. Numerous labels are known for such use in immunoassays. For example, compounds that may be detected directly, such as fluorochrome, chemiluminescent, and radioactive labels, as well as compounds that can be detected through reaction or derivitization, such as enzymes. Examples of these types of labels include ³²P, ¹⁴C, ¹²⁵I, ³H, and ¹³¹I radioisotopes, fluorophores such as rare earth chelates or fluorescein and its derivatives, rhodamine and its derivatives, dansyl, umbelliferone, luciferases such as firefly luciferase and bacterial luciferase (U.S. Patent No. 4,737,456), luciferin, 2,3-dihydrophthalavinediones, horseradish peroxidase (HRP), alkaline phosphatase, β-galactosidase, glucoamylase, lysozyme, saccharide oxidases such as glucose oxidase, galactose oxidase, and glucose-6-phosphate dehydrogenase. Heterocyclic oxidases such as uricase and xanthine oxidase, coupled to an enzyme that uses hydrogen peroxide to oxidize a dye precursor such as HRP, lactoperoxidase, or microperoxidase, biotin avidin, spin labels, bacteriophage labels, and stable free radicals.

The labels can be conjugated to dPNAG or anti-dPNAG antibody by methods known to those of ordinary skill in the art. For example, U.S. Patent Nos. 3,940,475 and 3,645,090 demonstrate conjugation of fluorophores and enzymes to antibodies. Other assays which reportedly are commonly used with antigen and antibody and which can be used include competition and sandwich assays.

The following Examples are included for purposes of illustration and are not intended to limit the scope of the invention.

### EXAMPLES

### Example 1: Expression of the bacterial surface polysaccharide poly-N-acetyl glucosamine (PNAG) by a variety of microbial species.

*Direct binding of antibodies:* Organisms are grown in various media to promote PNAG production at temperatures ranging from 20°C to 37°C for 24-72 hours in either atmospheric oxygen conditions (21% O₂), 5% CO₂ or under anaerobic conditions. Cells from plates are directly suspended into PBS (phosphate-buffered saline), while those in broth are washed and resuspended in PBS.

Samples are placed onto slides followed by methanol fixation for 1 min. Samples are further incubated with either MAb F429 (negative control, human IgG1 specific to Pseudomonas aeruginosa alginate antigen) directly conjugated to Alexafluor 488 (AF488) (1:313 dilution of 1.63 mg/ml stock, final concentration 5.2 ug/ml) or MAb F598 (human IgG1 specific to PNAG) directly conjugated to AF488 (1:833 dilution of 4.35 mg/ml stock, final concentration 5.2 ug/ml) in PBS containing 0.5% BSA overnight at 4°C and 4uM Syto 83. Samples are washed with PBS and mounted with 1.5 cover-glass for confocal analysis.

*Specificity of MAb F598 binding to PNAG:* Organisms are grown in various media to promote PNAG production at temperatures ranging from 20°C to 37°C for 24-72 hr in either atmospheric oxygen conditions (21% O₂), 5% CO₂ or under anaerobic conditions.. Bacterial cells from plates are directly suspended into TBS (tris-buffered saline, pH 6.5), while those in broth are washed and resuspended in TBS.

Generally, samples are treated with chitinase, Dispersin B or periodate and then exposed to MAb F598. Samples in TBS pH 6.5 are incubated with (a) 50 µg/ml chitinase (negative control), (b) Dispersin B (digests PNAG) for 24 hours at 37°C, or (c) with 0.4 M periodate for 2 hrs at 37°C. PNAG is cleaved by periodate, digested by Dispersin B, and unaffected by chitinase. Cells were then reacted with human IgG monoclonal antibody (MAb) to PNAG or IgG1 MAb to irrelevant antigen-*Pseudomonas aeruginosa* alginate (MAb F429 (negative control, human IgG1 specific to Pseudomonas aeruginosa alginate antigen). Antibody to the irrelevant antigen should not bind to PNAG.

Specifically, samples are washed and aliquots of 10 µl are air-dried onto glass slides, followed by methanol fixation for 1 min. MAb F429 (negative control) was directly conjugated to AF488 (1:313 dilution of 1.63 mg/ml stock, final concentration 5.2 µg/ml) and MAb F598 (anti-PNAG, lot 2) was directly conjugated to AF488 (1:833 dilution of 4.35 mg/ml stock, final concentration 5.2 ug/ml) in PBS containing 0.5% BSA overnight at 4°C along with 4 µM Syto 83 (stains DNA Red). Samples were washed with PBS and mounted onto glass slide with 1.5 coverglass for confocal analysis.

**Table 1**

| **Microbe** | **MAb F598** | **MAb F598 +Chitinase** | **MAb F598 + Dispersin** | **MAb F598 + Periodate** | **Negative Control** |
|---|---|---|---|---|---|
| *S. aureus (positive control)* | Positive (green) | Positive (green) | Negative (dark) | Negative (dark) | Negative (dark) |
| *Streptococcus pneumonia (strain D39)* | Positive (green) | Positive (green) | Negative (dark) | Negative (dark) | Negative (dark) |
| *Streptococcus pneumonia (strain ATCC8)* | Positive (green) | Positive (green) | Negative (dark) | Negative (dark) | Negative (dark) |
| *Streptococcus pyogenes (strain 950771)* | Positive (green) | Positive (green) | Negative (dark) | Negative (dark) | Negative (dark) |
| *Streptococcus agalactiae (strain M781)* | Positive (green) | Positive (green) | Negative (dark) | Negative (dark) | Negative (dark) |
| *Enterococcus faecalis (strain V583)* | Positive (green) | Positive (green) | Negative (dark) | ND | Negative (dark) |
| *Listeria monocytogenes* | Positive (green) | Positive (green) | Negative (dark) | Negative (dark) | Negative (dark) |
| *Clostridium difficile* | Positive (green) | Positive (green) | Negative (dark) | Negative (dark) | Negative (dark) |
| *Bacteroides fragilis (strain 9343)* | Positive (green) | Positive (green) | Negative (dark) | ND | Negative (dark) |
| *Mycobacterium tuberculosis (strain H37RV)* | Positive (green) | Positive (green) | Negative (dark) | Negative (dark) | Negative (dark) |
| *Mycobacterium smegmatis* | Positive (green) | Positive (green) | Negative (dark) | Negative (dark) | Negative (dark) |
| *Neisseria meningitides (strain B16B6)* | Positive (green) | Positive (green) | Negative (dark) | Negative (dark) | Negative (dark) |
| *Neisseria gonorrhoeae (strain 179008)* | Positive (green) | Positive (green) | Negative (dark) | Negative (dark) | Negative (dark) |
| *Hemophilus influenzae (non-typable) (strain 140)* | Positive (green) | Positive (green) | Negative (dark) | Negative (dark) | Negative (dark) |
| *Hemophilus ducreyi* | Positive (green) | Positive (green) | Negative (dark) | Negative (dark) | Negative (dark) |
| *Helicobacter pylori (strain 88-3857)* | Positive (green) | Positive (green) | Negative (dark) | Negative (dark) | Negative (dark) |
| *Campylobacter jejuni* | Positive (green) | Positive (green) | Negative (dark) | Negative (dark) | Negative (dark) |
| *Citrobacter rodentium* | Positive (green) | Positive (green) | Negative (dark) | ND | Negative (dark) |
| *Candida albicans (yeast)* | Positive (green) | Positive (green) | Negative (dark) | Negative (dark) | Negative (dark) |
| *Candida albicans (hyphae)* | Positive (green) | Positive (green) | Negative (dark) | Negative (dark) | Negative (dark) |
| *Aspergillus flavis* | Positive (green) | ND | ND | ND | Negative (dark) |
| *Fusarium* | Positive (green) | Positive (green) | Negative (dark) | Negative (dark) | Negative (dark) |
| *Cryptococcus neoformans* | Positive (green) | ND | ND | ND | Negative (dark) |
| *Plasmodium berghei (strain ANKA)-in infected mouse blood* | Positive (green) | Positive (green) | Negative (dark) | Negative (dark) | Negative (dark) |
| *Plasmodium falciparum (strain Senegal 2)* - *in infected blood* | Positive (green) | Positive (green) | Negative (dark) | Negative (dark) | Negative (dark) |
| *Salmonella enterica serovar typhi (strain TY2)* | Positive (green) | Positive (green) | Negative (dark) | Negative (dark) | Negative (dark) |
| *Salmonella enterica serovar typhimurium (strain LT2)* | Positive (green) | Positive (green) | Negative (dark) | Negative (dark) | Negative (dark) |

| | | | | | |
|---|---|---|---|---|---|
| ND = not done. | | | | | |

In addition, chitinase-resistant, dispersin B-sensitive PNAG was detected on infecting bacteria in samples of middle ear effusions (MEF) from children with *S. pneumoniae* otitis media and two MEF samples from children with nontypable *H. influenzae* otitis media. The same infecting bacteria also stained with either a *S. pneumoniae* or *H. influenzae*-specific antibody. PNAG was also detected in lung tissue from a *M. tuberculosis*-infected patient. In nasopharyngeal fluid from chinchillas experimentally infected with *S. pneumoniae* serogroup 19A, colocalization of the chitinase-resistant, dispersin B-sensitive PNAG antigen with the serogroup 19A capsule was readily seen. In the gastrointestinal tract of a mouse experimentally infected with *C*. *rodentium*, PNAG was detected around microbes associated with the epithelial cells. In ocular tissues from mice with *C*. *albicans* keratitis, PNAG was detected on the DNA-positive portion of the fungal cells.

In addition, bacterial strains reacting with MAb F598 but not with MAb F429 in confocal microscopic analysis include *Vibrio cholerae* 2R1 and *Vibrio cholerae* 0395.

Bacterial strains reacting with polyclonal rabbit antibody to PNAG but not with normal rabbit serum include *Bacteroides thetaiotamicron* and *Bacteroides vulgatis.*

### Example 2: C. rodentium in GI tract.

Samples were reacted with either MAb F429 (negative control, human IgG1 specific to *P. aeruginosa* alginate antigen) directly conjugated to AF488 (1:313 dilution of 1.63 mg/ml stock, final concentration 5.2 ug/ml) or MAb F598 (human IgG1 specific to PNAG) directly conjugated to AF488 (1:833 dilution of 4.35 mg/ml stock, final concentration 5.2 ug/ml) in PBS containing 0.5% BSA overnight at 4°C and 4 µM Syto 83 (for staining of nuclei). Samples are washed with PBS and mounted with 1.5 cover-glass for confocal analysis.

The results showed the presence of PNAG in *C*. *rodentium* colonies in the GI tract of an infected mouse using the F598 antibody. MAb F429 did not stain the colonies confirming the specificity of staining by the F598 MAb (data not shown).

### Example 3: S. pneumoniae in ear and nasal lavage fluid.

This Example demonstrates the co-expression on *S. pneumoniae* of capsule type 19A with PNAG antigen in the ear and nasal lavage fluid of a chinchilla with otitis media.

Samples were incubated with 50 µg/ml Dispersin B or chitinase in TBS overnight and then labeled with either 1:50 of F598 (1 mg/ml) or F429 (1 mg/ml) and rabbit anti-*S*. *pneumoniae* 19A capsular polysaccharide (1:100) in BSA/PBS overnight at 4°C followed by goat anti-human IgG conjugated to AF 488 and goat anti-rabbit AF 568 at 1:200 for 1 hour at room temperature.

The results showed the presence of PNAG surrounding the *S. pneumoniae* bacterial cells in nasopharyngeal washes of a chinchilla with otitis media. Samples treated with chitinase and Dispersin B and then exposed to control MAb F429 were negative. Samples treated with Dispersin B and then exposed to MAb F598 were negative, while samples treated with chitinase and then exposed to MAb F598 were positive, demonstrating the presence of PNAG in the nasopharyngeal wash of a chinchilla with otitis media. The staining patterns overlapped with rabbit anti-SPn 19A staining (data not shown).

Similarly, PNAG was detected in ear fluid (lavage) and nasal passages of a chinchilla infected with *S. pneumonia* 19A and having otitis media, as determined by staining with PNAG-specific MAb F598 but not with control MAb F429 following chitinase treatment. The staining patterns overlapped with rabbit anti-SPn 19A staining (data not shown).

### Example 4: S. pneumonia or non-typable H. influenza in human ear fluid from child with otitis media.

Ear fluid samples were incubated with 100 µg/ml DNaseI overnight at 37°C and then treated with 50 µg/ml chitinase or Dispersin B in TBS for 24 hours at 37°C. Samples were washed and aliquots of 10 µl were air-dried onto glass slides, heat fixed and labeled with 50 µg/ml of F598 (AP01) or F429 (1 mg/ml) and mouse anti-*S*. *pneumoniae* phosphatidylcholine (1:100) or guinea pig anti-*H*. *influenzae* (heat killed whole cells; 1:100) in PBS containing 0.5% BSA (PBS/BSA) overnight at 4°C. Samples were washed with PBS and incubated with 1:250 of anti-human IgG conjugated to AF488 and either 1:200 goat anti-mouse IgG or goat-anti guinea pig IgG conjugated to AF568 in PBS/BSA for 1 hour at room temperature. Slides were washed and mounted.

The results demonstrate the presence of PNAG in the ear fluid of the human subject having an *S. pneumoniae* ear infection (otitis media). Samples exposed to chitinase followed by MAb F429 or Dispersin B followed by MAb F598 were negative, while samples exposed to chitinase followed by MAb F598 were positive. The staining patterns overlapped with those observed with the mouse antibody to the *S. pneumoniae* phosphatidylcholine staining (data not shown).

### Example 5: P. berghei and P. falciparum in blood.

Confocal microscopy of unstained, fresh blood smear made from Malaria infected mice (BALB/CJ infected with *P. berghei* NK-65, Day 18 after infection). The smear was partitioned with a pap-pen into 3 wells for chitinase, Dispersin B, and periodate treatments. The slide was heat fixed and treated with 50 µg/ml chitinase or Dispersin B in TBS for 24 hours at 37°C or with 0.4 M periodate for 2 hours at 37°C. The slide was washed with PBS and incubated with MAb F598 (1.3 mg/ml stock; 1:250 dilution used, equal to 2.4 µl in 600 µl; final concentration 5.2 µg/ml or 3.12 µg in 600 µl; 200 µl added per well) directly conjugated to AF488 (green color). Also added was 4 µM of Syto 83 (DNA dye-red color) in PBS containing 0.5% BSA (BSA/PBS); both incubated for 4 hours at room temperature. The slide was washed with PBS and covered with 1.5 µm coverglass for viewing by confocal microscopy.

The results showed the presence of PNAG in the blood of a mouse that died from cerebral malaria. Samples treated with Dispersin B or periodate followed by staining with MAb F598 were negative, while samples treated with chitinase followed by staining with MAb F598 were positive (data now shown).

Using a similar approach, PNAG expression was analyzed in blood from a human infected with *P. falciparum.* Samples stained with control MAb F429 and samples treated with Dispersin B or periodate followed by staining with MAb F598 were negative (data not shown). Samples treated with chitinase or samples that were untreated, and then stained with F598 were positive (data not shown). The staining patterns of F598 overlapped with the identification of DNA inside of the red blood cells, which indicates the presence of the malaria parasite as human red blood cells do not contain a nucleus or DNA (data not shown).

### Example 6: B. dolosa in lung.

Using an approach similar to that described in the previous Examples, lung tissue from a cystic fibrosis patient who died of *B. dolosa* pneumonia and sepsis was sectioned and analyzed for PNAG expression. TO-PRO-3 was used to stain lung cell nuclei. *B. dolosa* cells were visualized using anti-*B*. *dolosa* mouse serum followed by AF568 donkey anti-mouse IgG. PNAG was visualized using rabbit antiserum to the 9GlcNH2-TT PNAG glycoform followed by AF488 goat anti-rabbit IgG. The negative control was normal rabbit serum with TO-PRO-3 nuclear stain. PNAG expression overlapped with *B. dolosa* cell staining (data not shown).

### Example 7: C. albicans in eyes.

Paraffin-embedded histology sections of *C*. *albicans* infected mouse eyes were deparaffinized using EzDewax as per manufacturer's instructions. Slides were rehydrated in water for 2 hours at room temperature, followed by blocking in 0.5% BSA/PBS for 2 hours at room temperature. Samples were further incubated with either MAb F429 (negative control) directly conjugated to AF488 (1:313 dilution of 1.63 mg/ml stock, final concentration 5.2 ug/ml) or MAb F598 to PNAG directly conjugated to AF488 (1:833 dilution of 4.35 mg/ml stock, final concentration 5.2 ug/ml) PBS containing 0.5% BSA overnight at 4°C and 4uM Syto 83. Samples were washed with PBS and mounted with 1.5 coverglass for confocal analysis.

The results showed confocal staining of nuclei with MAb F598 but not MAb F429, demonstrating expression of PNAG by *C*. *albicans* in vivo (data not shown).

### Example 8: Efficacy of MAb F598 to microbial PNAG in a murine model of C. albicans keratitis.

Keratitis was induced by scratching (3X 1 mm) the corneas of anesthetized male C57B1/6 mice followed by inoculation with ∼10¹-10⁷ CFU/eye of *Candida albicans* SC5314 in a 5 µl volume. PNAG-specific MAb (F598) or control human IgG1 MAb (F429) was delivered either by IP injection and/or topical application 4 h after infection and additional antibody applied topically 24 and 32 hours after infection. Mice were checked at 24 and 32 hours post-infection, and if controls showed irrecoverable corneal damage at this time point animals were euthanized and CFU/eye was determined. If controls at 36 hours had no more than 3+ damage to the damaged eye, the infection was allowed to progress to 48 hours, after which time mice were euthanized and CFU/eye and corneal pathology score of 0 to 4 were determined by observer without knowledge of mouse treatment. The corneal pathology score was as follows:
Score 4: Perforation of the cornea
Score 3: Dense opacity covering the anterior segment
Score 2: Dense opacity covering the pupil
Score 1: Faint opacity partially covering the pupil
Score 0: Identical to uninfected contralateral eye.

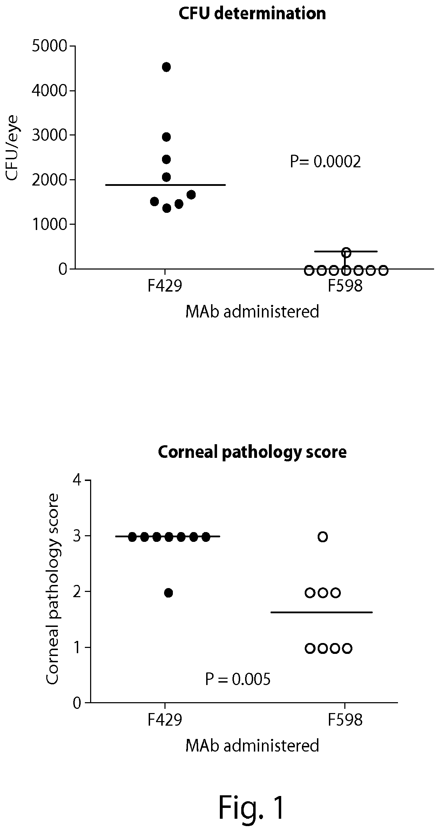

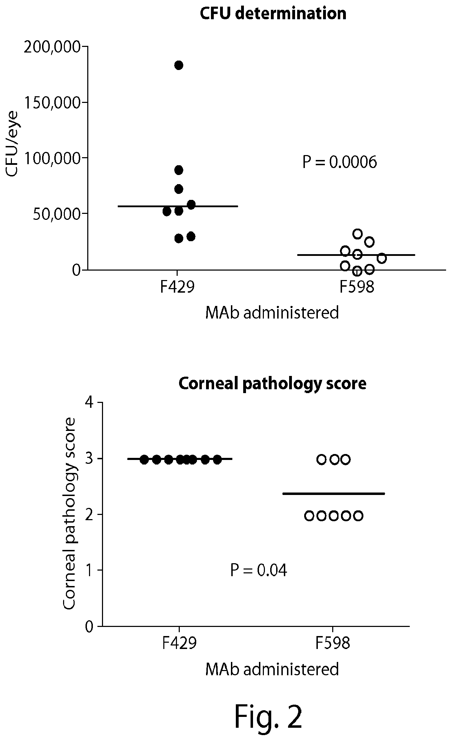

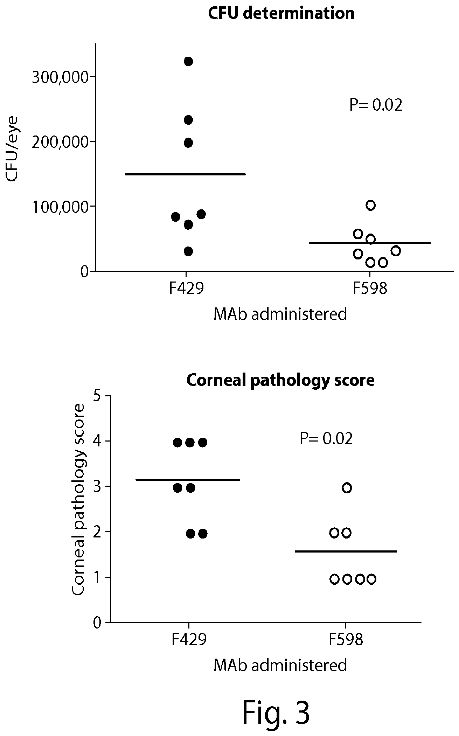

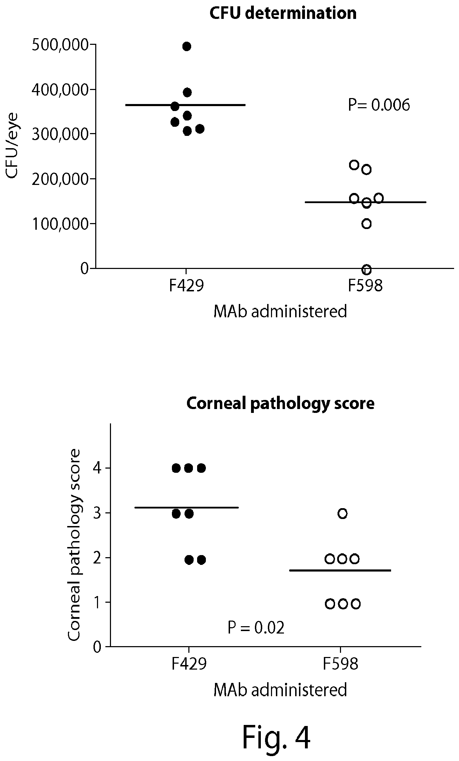

*C. albicans* infected mice treated therapeutically by IP injection and/or topical application of MAb F598 had markedly reduced bacterial levels in the eye and reduced corneal pathology compared to mice treated with control MAb F429. These data are presented in FIGs. 1-4.

Antibody to PNAG demonstrated therapeutic efficacy in a model of *C*. *albicans* keratitis, indicating in vivo expression of PNAG and the potential to prevent or treat fungal infections by vaccines and immunotherapeutics to PNAG.

### Example 9: Protective efficacy of polyclonal antibody to the 9GlcNH2-TT conjugate vaccine against lethal pneumonia caused by S. pneumonia.

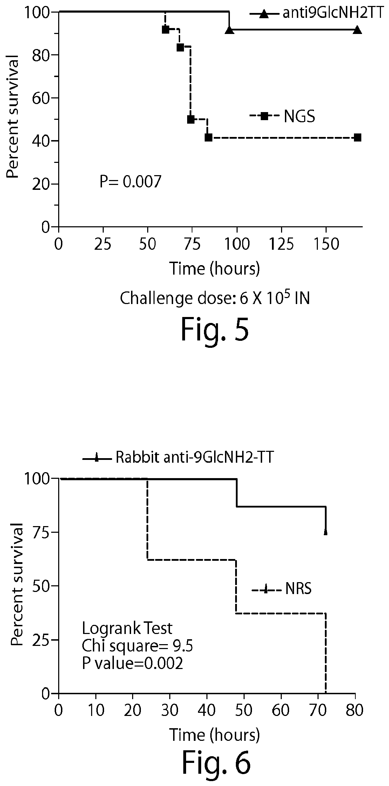

*S. pneumoniae* strain D39 was grown overnight in trypticase soy broth (TSB) at 37°C in 5% CO₂. Culture was diluted into Todd-Hewitt Broth + 1% glucose to an OD at 650 nm=0.1 and grown at 37°C until OD=0.45 (approximately 2.5 hours). CBA/N mice were injected retrorbitally (RO) under isoflurane anesthesia with 37.5 µl of heat-inactivated normal goat serum or goat antiserum raised to the anti-9GlcNH2-TT conjugate vaccine diluted to 50 µL in PBS. Mice were injected. Twenty four hours later, the bacterial preparation was diluted to OD at 650 nm = 0.45 (i.e., for this experiment, diluted 83 µL into 917 µL in PBS to give 3 x 107 CFU/mL. Mice were anesthetized with Ketamine/Xylazine (250µL per mouse), and 2 x 10 µL doses of bacteria were instilled intranasally. The mice were monitored for survival for 7 days. The results are shown in FIG. 5.

In addition, mAb F598 given 4 h before intranasal infection with serotype 9V DSM 11865 strain in FVB mice was found to be as potent as the antibiotic cefotaxime (administered at 1 and 4 h post-infection) in reducing bacterial burdens in the mouse lungs.

### Example 10: Protective efficacy of polyclonal antibody to the 9GlcNH2-TT conjugate vaccine against lethal skin infection caused by S. pyogenes (Group A Streptococcus).

Mice were CD1(ICR) 6-week old female mice. Group 1 (8 mice) corresponded to mice injected with normal rabbit sera (NRS). Group 2 (8 mice) corresponded to mice injected with 9GlcNH2-TT. Bacteria were Group A *Streptococcus* (GAS) strain 950771 used at 10⁷ CFU/ml in log phase. Antibodies were polyclonal rabbit anti-9GlcNH2-TT used at 200 µl/mouse/dose and NRS used at 200 µl/mouse/dose.

On day 0, a stock of GAS bacterial cells was streaked onto a blood agar plate (BAP) and incubated at 37°C overnight and the used to inoculate a tube of Todd-Hewitt Yeast Extract broth with 1% glucose (THY+G) for static culture at 37°C. Mice were injected IP 24 hour prior to infection. On day 1, mice were immunized a second time IP 4 hours prior to infection. To prepare the inoculum for infection, 10 ml of THY+G was used and a bacterial suspension at an OD₆₀₀nm=0.05 made and placed at 37°C until an OD₆₀₀nm = 0.15 was achieved. This was diluted and plated for CFU determinations on BAP as follows: 10 µl of the 10⁻⁵ and 10⁻⁶ dilutions were plated onto BAP (2 plates for each dilution). To prepare the infectious inoculum, 10 ml of the THY+G culture was transferred to a 15 ml conical tube and centrifuged the tube at 2000 rpm for 20 minutes to pellet the bacterial cells. The supernatant was discarded and the pellet suspended in 1 ml THY+G broth which was transferred to a microfuge tube and spun at 8000 rpm for 2 minutes. The supernatant was discarded and the pellet of bacterial cells was suspended in 500 µl injectable water, then transferred to a 1 ml 27 Gauge syringe, and kept on ice. A 1:4 dilution of 50 mg/ml Nembutal with injectable water [final conc 10 µg/ml] was made and injected IP injected (0.3 ml anesthetic solution per mouse). The right flank were shaved and swabbed with ethanol, then injected with 0.1 ml of the bacterial suspension subcutaneously per mouse (i.e., 5x10⁵ CFU/mouse). To confirm the actual inoculum, the bacterial suspension was diluted and plated for enumeration. The bacterial suspension injected had a concentration of 1.7x10⁶ CFU/ml, meaning 1.7x10⁵ CFU had been injected into each mouse. Mice were observed and moribund mice were euthanized. Results are shown in FIG. 6

### Example 11: Protective efficacy of antibody raised in rabbits to the 9GlcNH2-TT conjugate vaccine against meningitis (bacteria in the brain) of 2-3 day old mouse pups challenged with Group B N. meningitides.

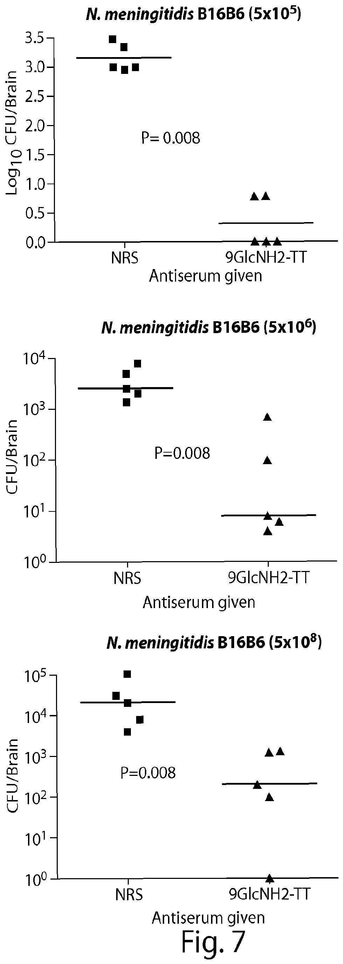

Mice were CD-1 neonatal mice on day 2 to day 3 of life. *N. meningitides* was grown on a blood agar plate overnight and bacterial cells were scraped into PBS and OD at 600 nm adjusted to give various challenge doses as noted. Mice were injected once 24 hour prior to infection with either NRS (50 µl IP (NRS from Accurate)) or rabbit serum raised to 9GlcNH₂-TT conjugate vaccine (50 µl IP (Rabbit 4.2, Bleed 4-3/4-4)). Mice were challenged 24 hours later IP with 50 µl of Serogroup B *N. meningitidis* strain B16B6 at the following dilutions: 5x10⁵, 5x10⁶ or 5x10⁸. Mice were sacrificed 24 hours later, and brains were removed, homogenized and plated on chocolate agar plates for bacterial enumeration. Results are shown in FIG. 7.

### Example 12: Protective efficacy of a fully human IgG1 MAb F598 when administered to mice susceptible to infectious colitis.

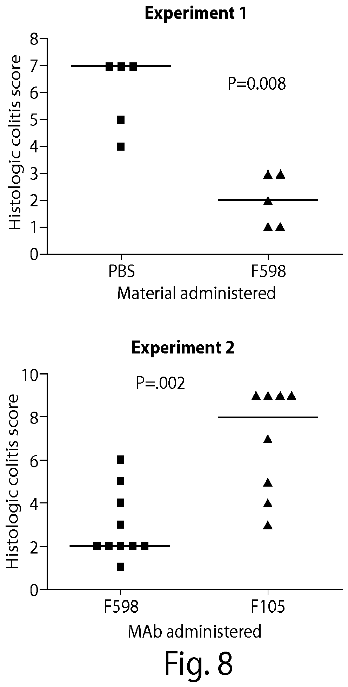

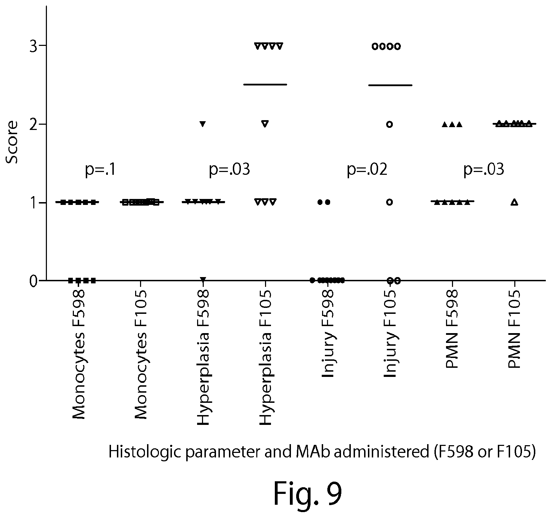

TRUC mice lack acquired and innate immune systems (Garrett et al. Cell 2007 131(1):33-45; Garrett et al. Cell Host Microbe, 2010 8(3):292-300). These mice spontaneously develop infectious colitis by 8 weeks of age. Newborn TRUC mice, starting in the first week of life, were injected ip with 50 µg of either MAb F598 or PBS, or MAb F598 or a control human IgG1 MAb, 3 times a week until sacrifice at 8 weeks. The intestinal tracts of the mice were removed and treated for evaluation of colitis as described (Garrett et al. Cell 2007 131(1):33-45; Garrett et al. Cell Host Microbe, 2010 8(3):292-300). The overall colitis pathology scores were compared between the groups of mice given either MAb F598 or control IgG1 MAb. The results are shown in FIGs. 8 and 9.

In other experiments, weekly administration of mAb F598 starting at day 7 of life significantly reduced the total histopathologic damage determined at 8 weeks of age (in a blinded fashion by a pathologist).

When WT neonates are cross-fostered by TRUC females, they develop spontaneous colitis at 8 weeks, although it is less severe than in TRUC offspring. To evaluate the therapeutic potential of mAb F598 against colitis in a setting of unperturbed immune system function, treatment of WT mice fostered by TRUC females was initiated at 4 weeks of age with biweekly injections of mAb F598 or control human IgG1 mAb F105 and the level of colonic pathology at 8 weeks of age was determined. mAb F598 significantly reduced the total pathology score in the recipient mice compared with controls, with significant reductions in monocyte infiltration and reactive hyperplasia, but not injury, because most of the controls had injury scores of zero (data not shown).

### Example 13: Protective efficacy of antibody raised in rabbits to the 9GlcNH2-TT conjugate vaccine against L. monocytogenes Strain 10403S.

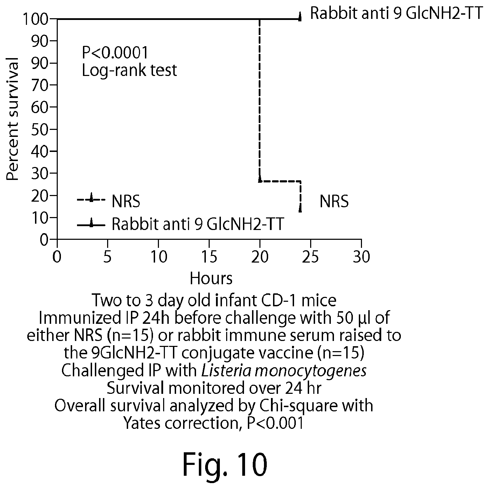

Two to three day old infant CD1 mice were immunized IP 24 hours before challenge with 50 µl of either NRS (n=15) or rabbit immune serum raised to the 9GlcNH2-TT conjugate vaccine (n=15). Mice were challenged IP with L. monocytogenes (% X 10^8 CFU in 50 ul). Survival was monitored over 24 hours. Overall survival was analyzed by Chi-square with Yates correction, P.001. The results are shown in FIG. 10.

An effective vaccine for malaria will likely require multiple parasite antigens, but as an initial step in determining if PNAG might be a candidate vaccine antigen component for a multivalent vaccine, C57BL/6 mice infected with PNAG-positive *P. berghei* ANKA were treated with 200 µL of polyclonal antibody to PNAG or control normal serum injected ip every 3 day starting the day before infection and through 20 days post-infection. Antibody to PNAG significantly extended the survival of the treated mice and prevented development of cerebral malaria. Five of eight mice treated with normal serum died by day 9 with low levels of parasitemia, and the remaining three died by day 30 with high levels of parasitemia. For the mice treated with antibody to PNAG for 3 weeks, only one died of cerebral malaria by day 7, four developed increasing levels of parasitemia and died by day 33, which was 13 days after the last injection of antibody, one had no detectable parasitemia until day 22 and died at day 40 (20 d after the last injection of antibody), and two mice had little to no detectable parasitemia and survived the 45-d experimental period. Antibody treatment was stopped at day 20 as per the initial protocol stipulation. It is likely that extended survival might be observed by increasing the duration of antibody treatment.

### Example 14: Antibody to PNAG mediates either opsonic or bactericidal killing of diverse PNAG-expressing pathogens.

The *S. pneumoniae* and *E. faecalis* opsonic killing protocol is similar to that published for *S. aureus* by Skurnik et al. (J Clin Invest. 2010, 120(9):3220-33) except that HL60 cells were used as the source of the phagocytic cells instead of fresh human PMN. HL60 cells were differentiated with 0.8% DMF for 6 days and adjusted to 1.3x10⁶/ml. Bacteria were grown overnight at 37°C (no shaking). *S. pneumonia* was placed in 5% CO₂ in THY +1% glucose (THY+G). *E. faecalis* was grown in atmospheric conditions. Bacterial strains were plated on TSB + blood agar at end of assay. Complement was adsorbed with target *S. aureus* MN8 bacteria grown overnight in CSB, shaking. C': Complement sera from Invitrogen Cat # 31203/S Lot # 510908698270 absorbed with S. aureus MN8 cells. Rabbit antibody was raised to one of two antigens:
1. Deacetylated PNAG (dPNAG) conjugated to tetanus toxoid (dPNAG-TT): Maira-Litran et al. Infect Immun. 2005, 73(10):6752-62. Erratum in: Infect Immun. 2005, 73(11):7789; and
2. Synthetic 9GlcNH2 oligosaccharide conjugated to TT: Gening et al. Infect Immun. 2010, 78(2):764-72.

Human MAb to PNAG, MAb F598, was also tested (Kelly-Quintos et al. Infect Immun. 2006, 74(5):2742-50).

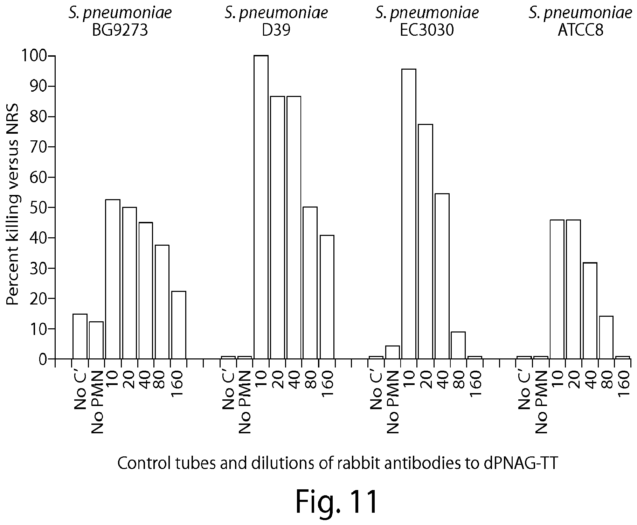

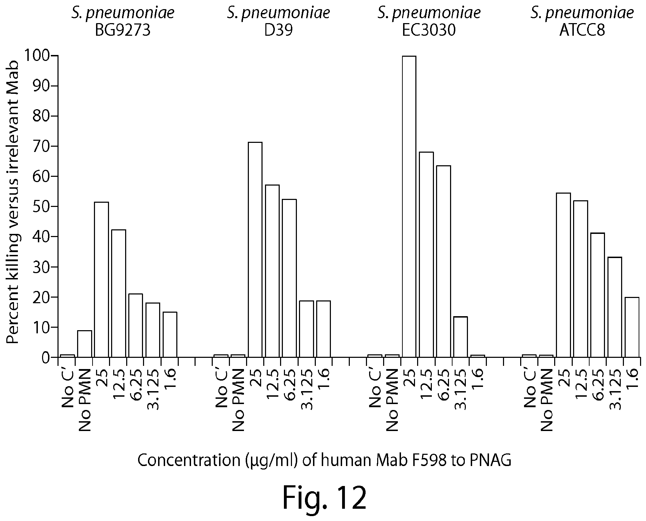

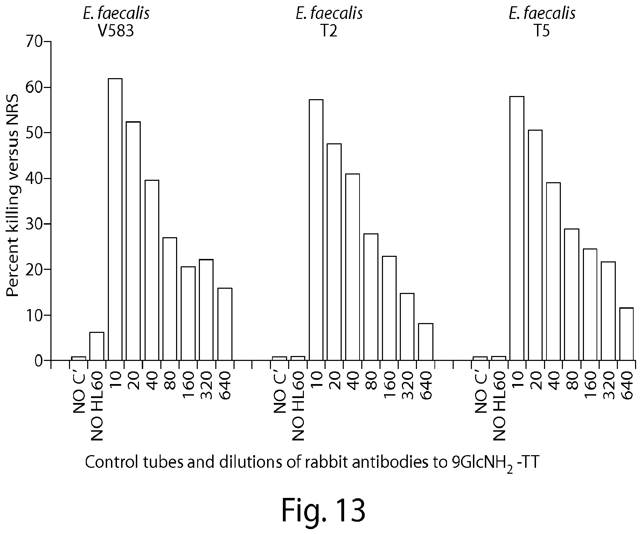

The results are shown in FIGs. 11-13.

The Group A *Streptococcus* opsonic killing protocol is as follows: Group A *Streptococcus* (GAS) strains 771, 188 were used. MAb lot used: F598 AP1-01 at [19 mg/ml], newly defrosted x1. Control for MAb F598: MAb F429 to *P. aeruginosa* alginate [1.0 mg/ml]. *S. aureus* MN8 strain was grown from frozen stock inoculated into 10 ml THB+G in 50ml conical at 37°C with loose lid shaking. GAS 771 and 188 were grown in THY+G from ON plate and inoculated at OD650=0.05 at 37°C static culture. *S. aureus* MN8 Dica was grown from frozen stock inoculated into in THB+G in10ml CSB in 50ml conical at 37C with loose lid shaking. GAS strains were plated on TSA with blood at end of assay. Complement was adsorbed with target *S. aureus MN8*Δ*ica* or GAS 771 bacteria grown overnight in THB+G with shaking or THY+G stat respectively. Buffer: HBSS + 0.1% Gelatin. C': Complement sera from Invitrogen Cat # 31203/S Lot # 510908698270 absorbed with cells of *S. aureus* MN8 *Dica* or GAS 771 or 188.

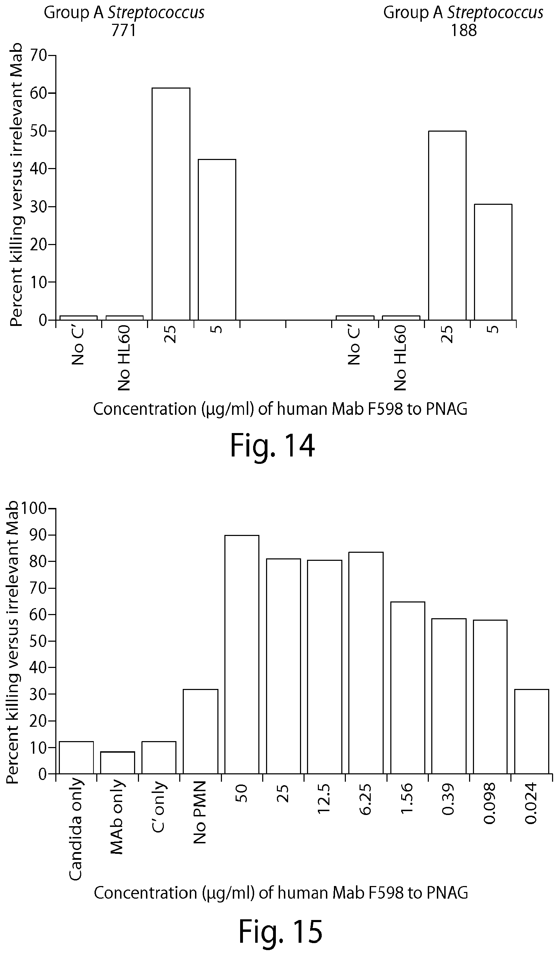

The results are shown in FIG. 14.

The C. *albicans* opsonic killing protocol is as follows: *C. albicans* was grown in YPD (Yeast extract/peptone/dextrose) broth to OD 1.0, then spun down, resuspended in MEM+1% BSA, OD adjusted to 0.4 at 650 nm then suspension diluted 1:10. MAb F429 was purified from hybridoma supernatant by Protein A and dialyzed into pH 6.5 PBS, stored -20°C. Buffer: MEM + 1% BSA. C': Complement sera from rabbit, Sigma Lot 106K6029, catalogue no. S7764. Complement was absorbed with C. *albicans* cells. Phagocytic cells were obtained from TRIMA collar donor.

The results are shown in FIG. 15.

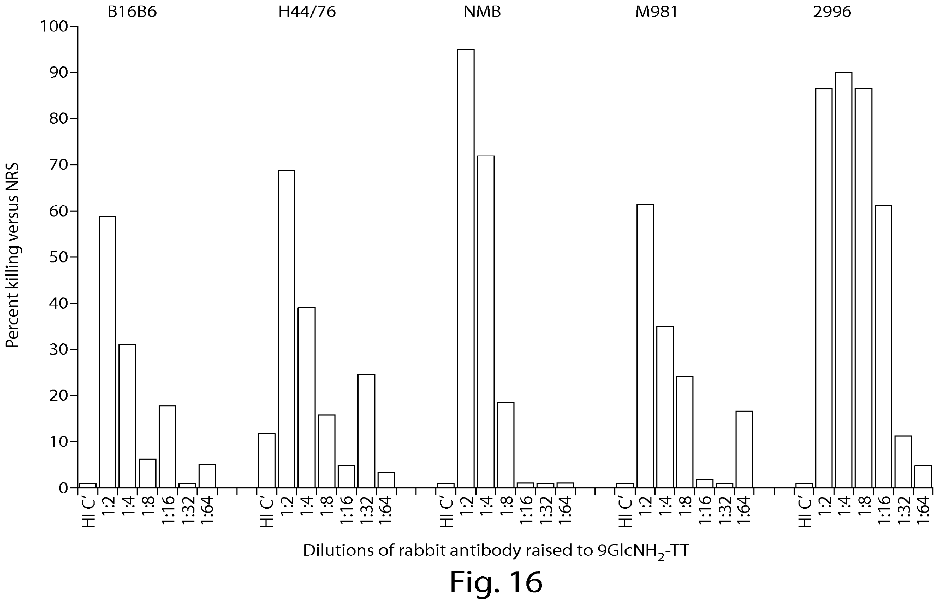

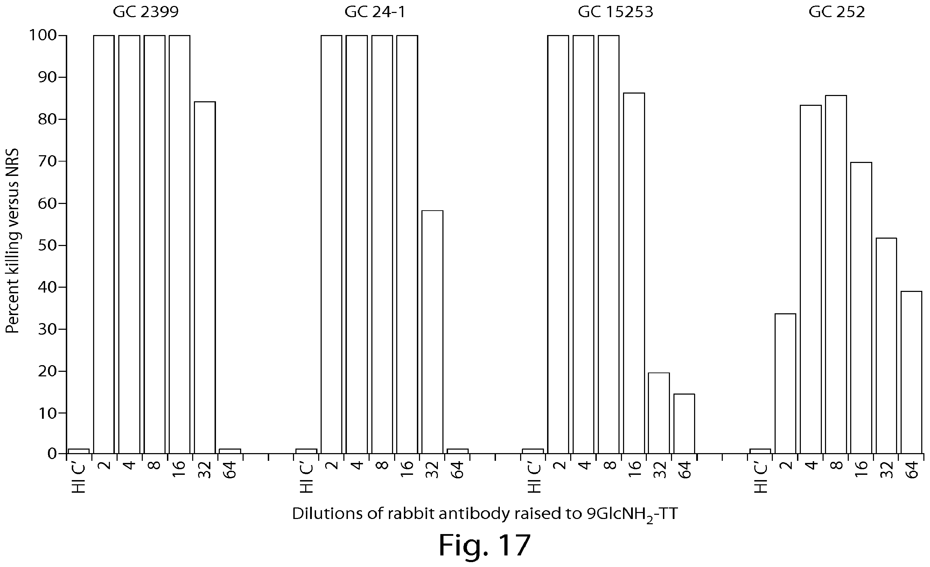

The *N. meningitidis* and *N. gonorrhoeae* bactericidal killing protocol is as follows: Normal Rabbit Serum (NRS from -20° stock, Accurate Chemical, lot 2008) and Rabbit antibody raised to 9GlcNH₂-TT conjugate vaccine, stored at -20°C, sera from rabbits 4.3 and 4.4 were used. The sera were equilibrated at 4°C and kept at 4°C until the assay. *N*. *meningitidis* or *N. gonorrhoeae* bacteria were grown for 24-48 hour on chocolate agar in 5% CO₂. Bacteria were suspended in PBS containing 0.5% glucose, 9 mM CaCl₂, and 4.9 mM MgCl₂·6H₂O and 1% (wt/vol) bovine serum albumin (Lifeblood Medical, Freehold, NJ)= PBS++, pH 7.4. Bacterial suspensions were diluted from the OD₆₅₀ nm suspension made from the chocolate agar suspension to ∼ 1.5-2 X 10⁴ CFU/ml in PBS++. The assay involved mixing together 80 µl of the serum dilutions and 20 µl of Neisserial cells then incubating the mixtures at 37°C for 1 hour. The tubes were placed at 37°C with mild shaking (orbital when possible). 10 µl of both the 1:2 and 10⁻¹ dilutions were made in Muller-Hinton Broth then plated onto chocolate agar. The cultures were incubated at 37°C in 5% CO₂ overnight. Colonies were counted the next day. The results are shown in FIGs. 16 and 17.

Bactericidal killing of *N. meningitidis* and *N. gonorrhoeae* strains in presence of rabbit serum to 9GlcNH₂-TT with and without specific antigen inhibition was carried out as follows:
Buffer: Buffer-PBS containing 0.5% glucose, 9 mM CaCl2, and 4.9 mM MgCl₂·6H₂O and 1% (wt/vol) bovine serum albumin (Lifeblood Medical, Freehold, NJ)= PBS++, pH 7.4.

Bacterial strains used and preparation: Resuspend the bacteria in PBS containing 0.5% glucose, 9 mM CaCl2, and 4.9 mM MgCl2·6H2O and 1% (wt/vol) bovine serum albumin (Lifeblood Medical, Freehold, NJ)= PBS++, pH 7.4. Dilute bacterial suspension from the OD650nm suspension from chocolate agar suspension of OD to ∼ 1.5-2 X 104 CFU/ml in PBS++.

Inhibiting antigens: Dissolve to 200 ug/ml final concentration in PBS++ PNAG antigen used: lot 27. Alginate antigen used from strain 2192, Pk 3 (run 1)

Antisera used: Normal Rabbit Serum (NRS from -20°C stock, Accurate Chemical). Rabbit antibody raised to 9GlcNH₂-TT conjugate vaccine, stored at -20°C, sera from rabbits 4.3 and 4.4 used.

Assay: Mix together 40 µl of the serum source (dilution based on the highest dilution in the direct bactericidal assay needed to achieve >80-90% killing, unless maximal killing at 1:2 serum dilution is lower, in which case use sera at 1:2 final dilution), 40 µl of antigen and 20 µl of Neisserial cells (tubes without antigen add 80 µl of properly-diluted antiserum). Place tubes at 37 C with mild shaking (orbital if possible). Plate on chocolate agar and incubate 37oC in 5% CO₂ overnight, count colonies the next day.

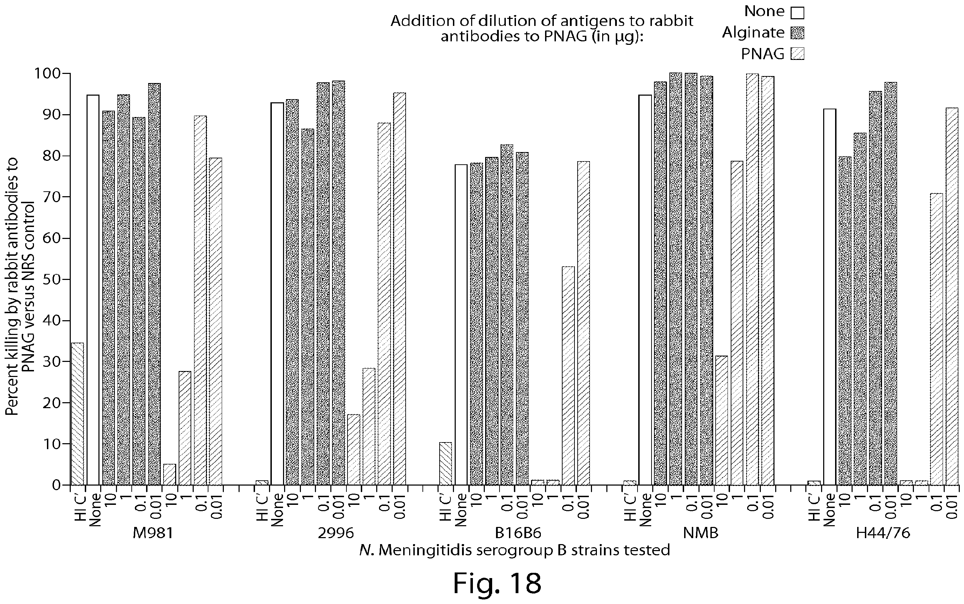

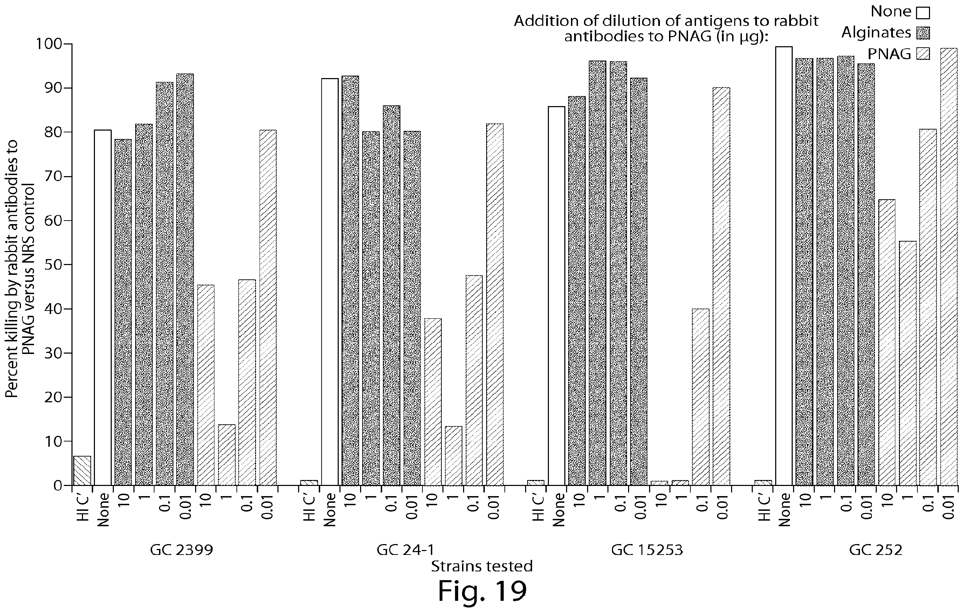

The results are shown in FIGs. 18 and 19.

### EQUIVALENTS

The foregoing written specification is considered to be sufficient to enable one skilled in the art to practice the invention. The present invention is not to be limited in scope by examples provided, since the examples are intended as a single illustration of one aspect of the invention and other functionally equivalent embodiments are within the scope of the invention. Various modifications of the invention in addition to those shown and described herein will become apparent to those skilled in the art from the foregoing description and fall within the scope of the appended claims. The advantages and objects of the invention are not necessarily encompassed by each embodiment of the invention.

## Claims

1. A pharmaceutical composition comprising an effective amount of an isolated polysaccharide conjugated to a carrier, the isolated polysaccharide having the formula 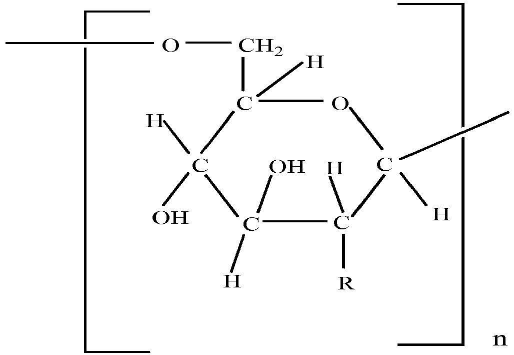 wherein n is at least 5, R is selected from the group consisting of -NH-CO-CH₃ and - NH₂, provided that less than 50% of the R groups are -NH-CQ-CH₃ for use in inducing an immune response against a pathogen in a subject having or at risk of developing an infection by the pathogen, wherein the pathogen is selected from:
(a) *S. pneumonia,* Group A *Streptococcus,* Group B *Streptococcus,* and *Enterococcus,*
(b) *Listeria*, *Clostridium difficile, B. subtilis, M. tuberculosis,* or *M*. *smegmatis,*
(c) *Neisseria meningitides, Neisseria gonorrhoeae,* Non-typable *H. influenzae, Helicobacter,* or *Campylobacter,*
(d) *Bacteroides fragilis, B. thetaiotamicron, B. vulgatis, Citrobacter rodentium, Vibrio cholera, Salmonella enterica* serovar typhi, or *Salmonella enterica* serovar typhimurium,
(e) *Candida albicans* (yeast), *Candida albicans* (hyphae), *Aspergillus, Fusarium,* or *Cryptococcus,*
(f) *P. bergei* or *P. falciparum,* and
(g) *Trichomonas vaginalis.*

2. The pharmaceutical composition for use according to claim 1, wherein the isolated polysaccharide is conjugated to the carrier through a linker.

3. The pharmaceutical composition for use according to claim 1 or claim 2, wherein
(a) less than 30%, less than 20%, less than 10%, or less than 5% of R groups are -NH-CO-CH₃, and/or
(b) none of the R groups is -NH-CO-CH₃, and/or
(c) n is at least 15, at least 20, at least 50, at least 100, at least 200, at least 300, at least 400 or at least 500, and/or
(d) the isolated polysaccharide has a molecular weight of 100-500 kDa.

4. The pharmaceutical composition for use according to any one of claims 1 to 3, wherein the isolated polysaccharide conjugated to the carrier is used with an adjuvant, and optionally formulated for
(a) systemic administration, or
(b) local administration.

5. A pharmaceutical composition comprising an effective amount of a PNAG- specific antibody or antigen-binding fragment thereof for use in inducing an immune response against a pathogen in a subject having or at risk of developing an infection by the pathogen, wherein the pathogen is selected from
(a) *S. pneumonia,* Group A *Streptococcus,* Group B *Streptococcus,* and *Enterococcus,*
(b) *Listeria, Clostridium difficile, B. subtilis, M. tuberculosis,* and *M*. *smegmatis,*
(c) *Neisseria meningitides, Neisseria gonorrhoeae,* Non-typable *H. influenzae, Helicobacter,* and *Campylobacter,*
(d) *Bacteroides fragilis, B. thetaiotamicron, B. vulgatis, Citrobacter rodentium, Vibrio cholera, Salmonella enterica* serovar typhi, and *Salmonella enterica* serovar typhimurium,
(e) *Candida albicans* (yeast), *Candida albicans* (hyphae), *Aspergillus, Fusarium,* and *Cryptococcus,*
(f) *P. bergei* and *P. falciparum,* and
(g) *Trichomonas vaginalis.*

6. The pharmaceutical composition for use according to claim 5, wherein the antibody or antigen-binding fragment thereofis formulated for
(a) systemic administration, or
(b) local administration,
optionally wherein the antibody or antibody fragment is
(a) ATCC PTA-5931 (F598) antibody or antigen-binding fragment thereof,
(b) ATCC PTA-5932 (F628) antibody or antigen-binding fragment thereof, or
(c) ATCC PTA-5933 (F630) antibody or antigen-binding fragment thereof,
further optionally wherein the antibody or antigen-binding fragment thereof is conjugated to an agent, optionally wherein the agent is a cytotoxic agent.

7. The pharmaceutical composition for use according to claim 5, wherein the antibody or antigen-binding fragment thereof is formulated for
(a) systemic administration, or
(b) local administration,
wherein the antibody or antibody fragment is ATCC PTA-5931 (F598) antibody or an antigen-binding fragment thereof.

8. The pharmaceutical composition for use according to any one of the preceding claims, wherein the subject is
(a) human, or
(b) a primate, horse, cow, swine, goat, sheep, dog, or cat.

9. The pharmaceutical composition for use according to any one of the preceding claims, wherein the subject
(a) has an infection by a pathogen of claim 1, or
(b) is at risk of developing an infection by a pathogen of claim 1.

10. The pharmaceutical composition for use according to any one of the preceding claims for use in preventing or treating infection by a pathogen recited in claim 1.

11. The pharmaceutical composition for use according to any one of claims 1-10, wherein the pathogen is selected from *Listeria*, *Clostridium difficile, B. subtilis, M. tuberculosis,* or M. *smegmatis.*

12. The pharmaceutical composition for use according to any one of claims 1-10, wherein the pathogen is *Neisseria meningitides, Neisseria gonorrhoeae,* Non-typable *H*. *Influenzae, Helicobacter,* or *Campylobacter.*

13. The pharmaceutical composition for use according to any one of claims 1-10, wherein the pathogen is *Bacteroides fragilis, B. thetaiotamicron, B. vulgatis, Citrobacter rodentium, Vibrio cholerae, Salmonella enterica* serovar typhi or *Salmonella enterica* serovar typhimurium.

14. The pharmaceutical composition for use according to any one of claims 1-10, wherein the pathogen is *Candida albicans* (yeast), *Candida albicans* (hyphae), *Aspergillus, Fusarium,* or *Cryptococcus.*

15. The pharmaceutical composition for use according to any one of claims 1-10, wherein the pathogen is *S*. *pneumonia.*

16. The pharmaceutical composition for use according to any one of claims 1-10, wherein the pathogen is Group A *Streptococcus.*

17. The pharmaceutical composition for use according to any of claims 1-10, wherein the pathogen is Group B *Streptococcus.*

18. The pharmaceutical composition for use according to any one of claims 1-10, wherein the pathogen is *M*. *tuberculosis.*

19. The pharmaceutical composition for use according to any one of claims 1-10, wherein the pathogen is *N. meningitidis.*

20. The pharmaceutical composition for use according to any one of claims 1-10, wherein the pathogen is *N. gonorrhoeae.*

21. The pharmaceutical composition for use according to any of claims 1-10, wherein the pathogen is *Salmonella enterica* serovar typhi.

22. The pharmaceutical composition for use according to any of claims 1-10, wherein the pathogen is *Candida albicans* (yeast).

23. The pharmaceutical composition for use according to any of claims 1-10, wherein the pathogen is *Candida albicans* (hyphae).

24. The pharmaceutical composition for use according to any of claims 1-10, wherein the pathogen is *Aspergillus.*

25. The pharmaceutical composition for use according to any of claims 1-10, wherein the pathogen is *Fusarium.*

26. The pharmaceutical composition for use according to any of claims 1-10, wherein the pathogen is *Cryptococcus.*

27. The pharmaceutical composition for use according to any one of claims 1-10, wherein the pathogen is *P. falciparum.*

28. The pharmaceutical composition for use according to any one of claims 1-4 and 8-27, wherein n is 5.

29. The pharmaceutical composition for use according to any one of claims 1-4 and 8-28, wherein the carrier is a peptide carrier.

30. The pharmaceutical composition for use according to any one of claims 1-4 and 8-29, wherein the carrier is tetanus toxoid.

31. The pharmaceutical composition for use according to any one of claims 1-4 and 8-30, wherein none of the R groups is -NH-CO-CH₃.

## Patentansprüche

1. Pharmazeutische Zusammensetzung, umfassend eine wirksame Menge eines isolierten Polysaccharids, das mit einem Träger konjugiert ist, wobei das isolierte Polysaccharid die Formel besitzt: 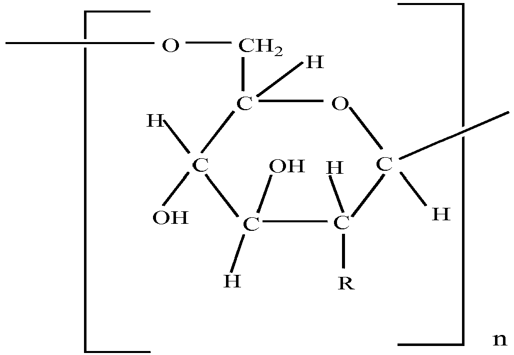 wobei n mindestens 5 ist, wobei R ausgewählt ist aus der Gruppe, bestehend aus - NH-CO-CH₃ und -NH₂, mit der Maßgabe, dass weniger als 50 % der R-Gruppen -NH-CO-CH₃ sind, zur Verwendung beim Induzieren einer Immunantwort gegen einen Erreger in einem Subjekt, das eine Infektion durch den Erreger hat oder gefährdet ist, eine Infektion durch den Erreger zu entwickeln, wobei der Erreger ausgewählt ist aus:
(a) *S*. *pneumonia, Streptococcus* der Gruppe A, *Streptococcus* der Gruppe B und *Enterococcus,*
(b) *Listeria, Clostridium difficile*, *B. subtilis, M. tuberculosis* oder *M. smegmatis,*
(c) *Neisseria meningitides, Neisseria gonorrhoeae,* nicht typisierbarer *H. influenzae, Helicobacter* oder *Campylobacter,*
(d) *Bacteroides fragilis, B. thetaiotamicron, B. vulgatis, Citrobacter rodentium, Vibrio cholera, Salmonella enterica* Serovar typhi oder *Salmonella enterica Serovar typhimurium,*
(e) *Candida albicans* (Hefe), *Candida albicans* (Hyphen), *Aspergillus, Fusarium,* oder *Cryptococcus,*
(f) *P. bergei* oder *P*. *falciparum,* und
(g) *Trichomonas vaginalis.*

2. Pharmazeutische Zusammensetzung zur Verwendung nach Anspruch 1, wobei das isolierte Polysaccharid über einen Linker an den Träger konjugiert ist.

3. Pharmazeutische Zusammensetzung zur Verwendung nach Anspruch 1 oder Anspruch 2, wobei
(a) weniger als 30 %, weniger als 20 %, weniger als 10 % oder weniger als 5 % der R-Gruppen -NH-CO-CH₃ sind, und/oder
(b) keine der R-Gruppen -NH-CO-CH₃ ist, und/oder
(c) n mindestens 15, mindestens 20, mindestens 50, mindestens 100, mindestens 200, mindestens 300, mindestens 400 oder mindestens 500 ist, und/oder
(d) das isolierte Polysaccharid ein Molekulargewicht von 100-500 kDa hat.

4. Pharmazeutische Zusammensetzung zur Verwendung nach einem der Ansprüche 1 bis 3, wobei das isolierte Polysaccharid, das mit dem Träger konjugiert ist, mit einem Adjuvans verwendet wird, und optional formuliert ist für eine
(a) systemische Verabreichung, oder
(b) lokale Verabreichung.

5. Pharmazeutische Zusammensetzung, umfassend eine wirksame Menge eines PNAGspezifischen Antikörpers oder eines antigen-bindenden Fragments davon zur Verwendung bei der Induktion einer Immunantwort gegen einen Erreger in einem Subjekt, das eine Infektion durch den Erreger hat oder gefährdet ist, eine Infektion durch den Erreger zu entwickeln, wobei der Erreger ausgewählt ist aus
(a) *S*. *pneumonia, Streptococcus* der Gruppe A, *Streptococcus* der Gruppe B und *Enterococcus,*
(b) *Listeria, Clostridium difficile*, *B. subtilis, M. tuberculosis* und *M. smegmatis,*
(c) *Neisseria meningitides, Neisseria gonorrhoeae,* nicht typisierbarer *H. influenzae, Helicobacter* und *Campylobacter,*
(d) *Bacteroides fragilis, B. thetaiotamicron, B. vulgatis, Citrobacter rodentium, Vibrio cholera, Salmonella enterica* Serovar typhi und *Salmonella enterica* Serovar typhimurium,
(e) *Candida albicans* (Hefe), *Candida albicans* (Hyphen), *Aspergillus, Fusarium* und *Cryptococcus,*
(f) *P. bergei* und *P*. *falciparum,* und
(g) *Trichomonas vaginalis.*

6. Pharmazeutische Zusammensetzung zur Verwendung nach Anspruch 5, wobei der Antikörper oder das antigenbindende Fragment davon formuliert ist für eine
(a) systemische Verabreichung, oder
(b) lokale Verabreichung,
optional, wobei der Antikörper oder das Antikörperfragment ist:
(a) ATCC-PTA-5931-(F598)-Antikörper oder antigenbindendes Fragment davon,
(b) ATCC-PTA-5932-(F628)-Antikörper oder antigenbindendes Fragment davon, oder
(c) ATCC-PTA-5933-(F630)-Antikörper oder antigenbindendes Fragment davon,
wobei ferner optional der Antikörper oder das antigenbindende Fragment davon mit einem Mittel konjugiert ist, wobei das Mittel optional ein zytotoxisches Mittel ist.

7. Pharmazeutische Zusammensetzung zur Verwendung nach Anspruch 5, wobei der Antikörper oder das antigenbindende Fragment davon formuliert ist für eine
(a) systemische Verabreichung, oder
(b) lokale Verabreichung,
wobei der Antikörper oder das Antikörperfragment ein ATCC-PTA-5931-(F598)-Antikörper oder ein antigenbindendes Fragment davon ist.

8. Pharmazeutische Zusammensetzung zur Verwendung nach einem der vorhergehenden Ansprüche, wobei das Subjekt ist:
(a) ein Mensch, oder
(b) ein Primat, ein Pferd, ein Rind, ein Schwein, eine Ziege, ein Schaf, ein Hund oder eine Katze.

9. Pharmazeutische Zusammensetzung zur Verwendung nach einem der vorhergehenden Ansprüche, wobei das Subjekt:
(a) eine Infektion durch einen Erreger nach Anspruch 1 hat, oder
(b) gefährdet ist, eine Infektion durch einen Erreger nach Anspruch 1 zu entwickeln.

10. Pharmazeutische Zusammensetzung zur Verwendung nach einem der vorhergehenden Ansprüche zur Verwendung bei der Prävention oder Behandlung von Infektionen durch einen Erreger nach Anspruch 1.

11. Pharmazeutische Zusammensetzung zur Verwendung nach einem der Ansprüche 1 bis 10, wobei der Erreger ausgewählt ist aus *Listeria, Clostridium difficile, B. subtilis, M. tuberculosis* oder *M. smegmati.*

12. Pharmazeutische Zusammensetzung zur Verwendung nach einem der Ansprüche 1 bis 10, wobei der Erreger *Neisseria meningitides, Neisseria gonorrhoeae,* nicht typisierbare *H. Influenzae, Helicobacter* oder *Campylobacter* ist.

13. Pharmazeutische Zusammensetzung zur Verwendung nach einem der Ansprüche 1 bis 10, wobei der Erreger *Bacteroides fragilis, B. thetaiotamicron, B. vulgatis, Citrobacter rodentium, Vibrio cholerae, Salmonella enterica* Serovar typhi oder *Salmonella enterica* Serovar typhimurium ist.

14. Pharmazeutische Zusammensetzung zur Verwendung nach einem der Ansprüche 1 bis 10, wobei der Erreger *Candida albicans* (Hefe), *Candida albicans* (Hyphen), *Aspergillus, Fusarium* oder *Cryptococcus* ist.

15. Pharmazeutische Zusammensetzung zur Verwendung nach einem der Ansprüche 1 bis 10, wobei der Erreger *S. pneumonia* ist.

16. Pharmazeutische Zusammensetzung zur Verwendung nach einem der Ansprüche 1 bis 10, wobei der Erreger *Streptococcus* der Gruppe A ist.

17. Pharmazeutische Zusammensetzung zur Verwendung nach einem der Ansprüche 1 bis 10, wobei der Erreger *Streptococcs* der Gruppe B ist.

18. Pharmazeutische Zusammensetzung zur Verwendung nach einem der Ansprüche 1 bis 10, wobei der Erreger *M. tuberculosis* ist.

19. Pharmazeutische Zusammensetzung zur Verwendung nach einem der Ansprüche 1 bis 10, wobei der Erreger *N. meningitidis* ist.

20. Pharmazeutische Zusammensetzung zur Verwendung nach einem der Ansprüche 1 bis 10, wobei der Erreger *N. gonorrhoeae* ist.

21. Pharmazeutische Zusammensetzung zur Verwendung nach einem der Ansprüche 1 bis 10, wobei der Erreger *Salmonella enterica* Serovar typhi ist.

22. Pharmazeutische Zusammensetzung zur Verwendung nach einem der Ansprüche 1 bis 10, wobei der Erreger *Candida albicans* (Hefe) ist.

23. Pharmazeutische Zusammensetzung zur Verwendung nach einem der Ansprüche 1 bis 10, wobei der Erreger *Candida albicans* (Hyphen) ist.

24. Pharmazeutische Zusammensetzung zur Verwendung nach einem der Ansprüche 1 bis 10, wobei der Erreger *Aspergillus* ist.

25. Pharmazeutische Zusammensetzung zur Verwendung nach einem der Ansprüche 1 bis 10, wobei der Erreger *Fusarium* ist.

26. Pharmazeutische Zusammensetzung zur Verwendung nach einem der Ansprüche 1 bis 10, wobei der Erreger *Cryptococcus* ist.

27. Pharmazeutische Zusammensetzung zur Verwendung nach einem der Ansprüche 1 bis 10, wobei der Erreger *P. falciparum* ist.

28. Pharmazeutische Zusammensetzung zur Verwendung nach einem der Ansprüche 1 bis 4 und 8 bis 27, wobei n 5 ist.

29. Pharmazeutische Zusammensetzung zur Verwendung nach einem der Ansprüche 1 bis 4 und 8 bis 28, wobei der Träger ein Peptidträger ist.

30. Pharmazeutische Zusammensetzung zur Verwendung nach einem der Ansprüche 1 bis 4 und 8 bis 29, wobei der Träger ein Tetanustoxoid ist.

31. Pharmazeutische Zusammensetzung zur Verwendung nach einem der Ansprüche 1 bis 4 und 8 bis 30, wobei keine der R-Gruppen -NH-CO-CH₃ ist.

## Revendications

1. Composition pharmaceutique comprenant une quantité efficace d'un polysaccharide isolé conjugué à un support, le polysaccharide isolé ayant la formule 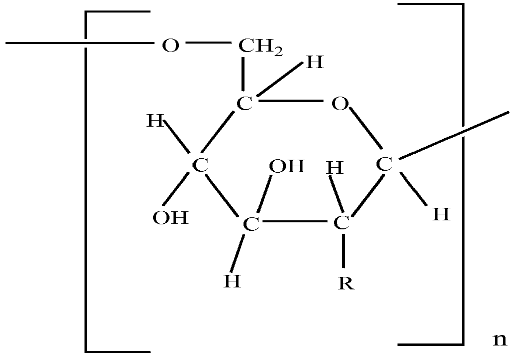 dans laquelle n est au moins 5, R est choisi dans le groupe constitué de -NH-CO-CH₃ et - NH₂, à condition que moins de 50 % des groupes R soient -NH-CO-CH₃ à utiliser dans l'induction d'une réponse immunitaire contre un agent pathogène chez un sujet présentant ou risquant de développer une infection par l'agent pathogène, l'agent pathogène étant choisi parmi:
(a) *S*. *pneumonia, Streptococcus* Groupe *A, Streptococcus* Groupe B et *Enterococcus,*
(b) *Listeria, Clostridium difficile, B. subtilis, M. tuberculosis* ou *M. smegmatis,*
(c) *Neisseria meningitides, Neisseria gonorrhoeae, H. influenzae* non typable, *Helicobacter* ou *Campylobacter,*
(d) *Bacteroides fragilis, B.thetaiotamicron, B. vulgatis, Citrobacter rodentium, Vibrio choiera, Salmonella enterica* serovar typhi ou *Salmonella enterica* serovar typhimurium,
(e) *Candida albicans* (levure), *Candida albicans* (hyphes), *Aspergillus, Fusarium* ou *Cryptococcus,*
(f) *P. bergei* ou *P*. *falciparum,* et
(g) *Trichomonas vaginalis.*

2. Composition pharmaceutique destinée à être utilisée selon la revendication 1, dans laquelle le polysaccharide isolé est conjugué au support par l'intermédiaire d'un lieur.

3. Composition pharmaceutique destinée à être utilisée selon la revendication 1 ou 2, dans laquelle
(a) moins de 30 %, moins de 20 %, moins de 10 % ou moins de 5 % des groupes R sont - NH- CO-CH₃, et/ou
(b) aucun des groupes R n'est -NH-CO-CH₃, et/ou
(c) n est au moins 15, au moins 20, au moins 50, au moins 100, au moins 200, au moins 300, au moins 400 ou au moins 500, et/ou
(d) le polysaccharide isolé a un poids moléculaire de 100 à 500 kDa.

4. Composition pharmaceutique destinée à être utilisée selon l'une quelconque des revendications 1 à 3, dans laquelle le polysaccharide isolé conjugué au support est utilisé avec un adjuvant, et facultativement formulé pour
(a) administration systémique, ou
(b) administration locale.

5. Composition pharmaceutique comprenant une quantité efficace d'un anticorps spécifique de PNAG ou d'un fragment de liaison à l'antigène de celui-ci destinée à être utilisée dans l'induction d'une réponse immunitaire contre un agent pathogène chez un sujet présentant ou risquant de développer une infection par l'agent pathogène, l'agent pathogène étant choisi parmi
(a) *S. pneumonia, Streptococcus* Groupe *A, Streptococcus* Groupe B et *Enterococcus,*
(b) *Listeria, Clostridium difficile, B. subtilis, M. tuberculosis* et *M. smegmatis,*
(c) *Neisseria meningitides, Neisseria gonorrhoeae, H. influenzae* non typable, *Helicobacter* et *Campylobacter,*
*(d) Bacteroides fragilis, B.thetaiotamicron, B. vulgatis, Citrobacter rodentium, Vibrio cholera, Salmonella enterica* serovar typhi et *Salmonella enterica* serovar typhimurium,
(e) *Candida albicans* (levure), *Candida albicans* (hyphes), *Aspergillus, Fusarium* et *Cryptococcus,*
(f) *P. bergei* et *P*. *falciparum,* et
(g) *Trichomonas vaginalis.*

6. Composition pharmaceutique destinée à être utilisée selon la revendication 5, dans laquelle l'anticorps ou le fragment de liaison à l'antigène de celui-ci est formulé pour
(a) administration systémique, ou
(b) administration locale,
facultativement dans laquelle l'anticorps ou le fragment d'anticorps est
(a) l'anticorps ATCC PTA-5931 (F598) ou le fragment de liaison à l'antigène de celui-ci,
(b) l'anticorps ATCC PTA-5932 (F628) ou le fragment de liaison à l'antigène de celui-ci, ou
(c) l'anticorps ATCC PTA-5933 (F630) ou le fragment de liaison à l'antigène de celui-ci,
en outre facultativement l'anticorps ou le fragment de liaison à l'antigène de celui-ci étant conjugué à un agent, facultativement dans laquelle l'agent est un agent cytotoxique.

7. Composition pharmaceutique destinée à être utilisée selon la revendication 5, dans laquelle l'anticorps ou le fragment de liaison à l'antigène de celui-ci est formulé pour
(a) administration systémique, ou
(b) administration locale,
l'anticorps ou le fragment d'anticorps étant l'anticorps ATCC PTA-5931 (F598) ou un fragment de liaison à un antigène de celui-ci.

8. Composition pharmaceutique destinée à être utilisée selon l'une quelconque des revendications précédentes, dans laquelle le sujet est
(a) humain, ou
(b) un primate, un cheval, une vache, un porc, une chèvre, un mouton, un chien ou un chat.

9. Composition pharmaceutique destinée à être utilisée selon l'une quelconque des revendications précédentes, dans laquelle le sujet
(a) présente une infection par un agent pathogène de la revendication 1, ou
(b) risque de développer une infection par un agent pathogène de la revendication 1.

10. Composition pharmaceutique destinée à être utilisée selon l'une quelconque des revendications précédentes, destinée à être utilisée dans la prévention ou le traitement d'une infection par un agent pathogène selon la revendication 1.

11. Composition pharmaceutique destinée à être utilisée selon l'une quelconque des revendications 1 à 10, dans laquelle l'agent pathogène est choisi parmi *Listeria, Clostridium difficile, B. subtilis, M. tuberculosis* ou *M. smegmatis.*

12. Composition pharmaceutique destinée à être utilisée selon l'une quelconque des revendications 1 à 10, dans laquelle l'agent pathogène est *Neisseria meningitides, Neisseria gonorrhoeae, H. Influenzae* non typable, *Helicobacter* ou *Campylobacter.*

13. Composition pharmaceutique destinée à être utilisée selon l'une quelconque des revendications 1 à 10, dans laquelle l'agent pathogène est *Bacteroides fragilis, B. thetaiotamicron, B. vulgatis, Citrobacter rodentium, Vibrio cholerae, Salmonella enterica* serovar typhi ou *Salmonella enterica* serovar typhimurium.

14. Composition pharmaceutique destinée à être utilisée selon l'une quelconque des revendications 1 à 10, dans laquelle l'agent pathogène est *Candida albicans* (levure), *Candida albicans* (hyphes), *Aspergillus, Fusarium* ou *Cryptococcus.*

15. Composition pharmaceutique destinée à être utilisée selon l'une quelconque des revendications 1 à 10, dans laquelle l'agent pathogène est *S. pneumonia.*

16. Composition pharmaceutique destinée à être utilisée selon l'une quelconque des revendications 1 à 10, dans laquelle l'agent pathogène est le *streptocoque* Groupe A.

17. Composition pharmaceutique destinée à être utilisée selon l'une quelconque des revendications 1 à 10, dans laquelle l'agent pathogène est le *streptocoque* Groupe B.

18. Composition pharmaceutique destinée à être utilisée selon l'une quelconque des revendications 1 à 10, dans laquelle l'agent pathogène est *M. tuberculosis.*

19. Composition pharmaceutique destinée à être utilisée selon l'une quelconque des revendications 1 à 10, dans laquelle l'agent pathogène est *N. meningitidis.*

20. Composition pharmaceutique destinée à être utilisée selon l'une quelconque des revendications 1 à 10, dans laquelle l'agent pathogène est *N. gonorrhoeae.*

21. Composition pharmaceutique destinée à être utilisée selon l'une quelconque des revendications 1 à 10, dans laquelle le pathogène est *Salmonella enterica* serovar typhi.

22. Composition pharmaceutique destinée à être utilisée selon l'une quelconque des revendications 1 à 10, dans laquelle l'agent pathogène est *Candida albicans* (levure).

23. Composition pharmaceutique destinée à être utilisée selon l'une quelconque des revendications 1 à 10, dans laquelle l'agent pathogène est *Candida albicans* (hyphes).

24. Composition pharmaceutique destinée à être utilisée selon l'une quelconque des revendications 1 à 10, dans laquelle l'agent pathogène est *Aspergillus.*

25. Composition pharmaceutique destinée à être utilisée selon l'une quelconque des revendications 1 à 10, dans laquelle l'agent pathogène est le *fusarium.*

26. Composition pharmaceutique destinée à être utilisée selon l'une quelconque des revendications 1 à 10, dans laquelle l'agent pathogène est *Cryptococcus.*

27. Composition pharmaceutique destinée à être utilisée selon l'une quelconque des revendications 1 à 10, dans laquelle l'agent pathogène est *P*. *falciparum.*

28. Composition pharmaceutique destinée à être utilisée selon l'une quelconque des revendications 1 à 4 et 8 à 27, dans laquelle n est 5.

29. Composition pharmaceutique destinée à être utilisée selon l'une quelconque des revendications 1 à 4 et 8 à 28, dans laquelle le support est un support peptidique.

30. Composition pharmaceutique destinée à être utilisée selon l'une quelconque des revendications 1 à 4 et 8 à 29, dans laquelle le support est l'anatoxine tétanique.

31. Composition pharmaceutique destinée à être utilisée selon l'une quelconque des revendications 1 à 4 et 8 à 30, dans laquelle aucun des groupes R n'est -NH-CO-CH₃.
